(19) Europäisches Patentamt / European Patent Office / Office européen des brevets

(11) **EP 4 332 117 A1**

(12) **EUROPEAN PATENT APPLICATION**
published in accordance with Art. 153(4) EPC

(43) Date of publication:
**06.03.2024 Bulletin 2024/10**

(21) Application number: **22794876.7**

(22) Date of filing: **26.04.2022**

(51) International Patent Classification (IPC):
***C07K 16/28*** *(2006.01)* ***C12N 15/13*** *(2006.01)*
***A61K 39/395*** *(2006.01)* ***A61K 47/68*** *(2017.01)*
***A61K 31/4745*** *(2006.01)* ***A61P 35/00*** *(2006.01)*

(52) Cooperative Patent Classification (CPC):
**A61K 47/6855; A61K 47/68; A61K 47/68037; A61P 35/00; C07K 16/2803;** A61K 2039/505; C07K 2317/21; C07K 2317/77; C07K 2317/92

(86) International application number:
**PCT/CN2022/089129**

(87) International publication number:
**WO 2022/228406 (03.11.2022 Gazette 2022/44)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **26.04.2021 CN 202110455570**

(71) Applicants:
- **Jiangsu Hengrui Pharmaceuticals Co., Ltd.**
  **Lianyungang, Jiangsu 222047 (CN)**
- **Shanghai Hengrui Pharmaceutical Co., Ltd.**
  **Shanghai 200245 (CN)**

(72) Inventors:
- **YANG, Yang**
  **Shanghai 200245 (CN)**
- **ZHANG, Haoying**
  **Shanghai 200245 (CN)**
- **TAO, Weikang**
  **Shanghai 200245 (CN)**

(74) Representative: **Regimbeau**
**20, rue de Chazelles**
**75847 Paris Cedex 17 (FR)**

Remarks:
The complete document including Reference Table(s) and the Sequence Listing(s) can be downloaded from the EPO website

# (54) ANTI-NECTIN-4 ANTIBODY AND ANTI-NECTIN-4 ANTIBODY-DRUG CONJUGATE, AND MEDICINAL USER THEREOF

(57) Provided are an anti-Nectin-4 antibody and an anti-Nectin-4 antibody-drug conjugate, and a medicinal user thereof. The anti-Nectin-4 antibody, and the anti-Nectin-4 antibody-drug conjugate as represented by general formula (Pc-L-Y-D) are involved, wherein Pc is the anti-Nectin-4 antibody, and L, Y, and n are as defined in the description.

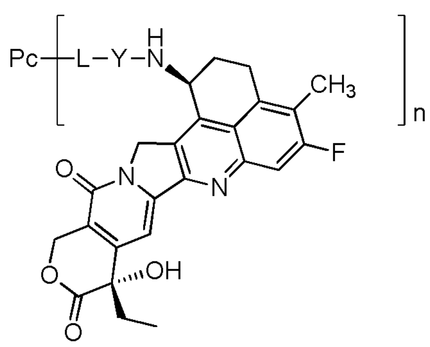

(Pc-L-Y-D)

EP 4 332 117 A1

## Description

### TECHNICAL FIELD

The present disclosure relates to an anti-Nectin-4 antibody and an anti-Nectin-4 antibody-exatecan analog conjugate, a preparation method therefor, a pharmaceutical composition comprising same, and use thereof in the preparation of a medicament for treating a Nectin-4-mediated disease or disorder, particularly in the preparation of an anti-cancer medicament.

### BACKGROUND

The statements herein merely provide background information related to the present disclosure and may not necessarily constitute the prior art.

Nectin-4 (PVRL4) is a 66 kDa type I transmembrane glycoprotein and belongs to the Nectin family of the Ig superfamily. It plays a key role in various biological processes (proliferation, differentiation and migration) of epithelial, endothelial, immune and neural cells. Research shows that Nectin-4 plays an important role in the development, infiltration and metastasis of malignant tumor tissues such as bladder cancer, breast cancer and lung cancer. This role is associated with the activation of Cdc42 and Rac and the alteration of cytoskeletal actin and causes the proliferation, infiltration and metastasis of tumor cells.

Nectin-4 is a tumor-specifically expressed antigen. It is expressed in 50% of bladder cancer, 49% of breast cancer and 86% of lung cancer and is frequently found in tumors with poor prognosis, but its expression is not found in most normal tissues. The above information suggests that Nectin-4 can be an ideal target for tumor treatment.

The enfortumab vedotin (Padcev) disclosed in WO2012047724 is a Nectin-4-targeting conjugate coupled to MMAE. It can be used for treating adult locally advanced or metastatic urothelial cancer in patients who have previously received platinum-containing chemotherapy and PD-1/L1 inhibitor treatment (European Commission Approves PADCEV (enfortumab vedotin) for Locally Advanced or Metastatic Urothelial Cancer. Press release, Seagen Inc; 04/13/2022. Accessed 04/14/2022). More antibody-drug conjugates taking Nectin-4 as a target have great significance as anti-tumor drugs for research.

### SUMMARY

The present disclosure relates to an anti-Nectin-4 antibody, an anti-Nectin-4 antibody-exatecan analog conjugate and use thereof.

The present disclosure provides an anti-Nectin-4 antibody, wherein the anti-Nectin-4 antibody binds to Nectin-4 protein expressed by T47D cells or MDA-MB-468 cells with an $EC_{50}$ value of less than 0.1 nM, as determined by FACS.

In some embodiments, the anti-Nectin-4 antibody according to any one of the above comprises a HCDR1, a HCDR2 and a HCDR3 contained in a heavy chain variable region set forth in SEQ ID NO: 6; and a LCDR1, a LCDR2 and a LCDR3 contained in a light chain variable region set forth in SEQ ID NO: 7.

In some embodiments, the anti-Nectin-4 antibody according to any one of the above comprises a heavy chain variable region and a light chain variable region, wherein:

a. the heavy chain variable region comprises a HCDR1, a HCDR2 and a HCDR3 set forth in SEQ ID NO: 8, SEQ ID NO: 9 and SEQ ID NO: 10, respectively; and the light chain variable region comprises a LCDR1, a LCDR2 and a LCDR3 set forth in SEQ ID NO: 11, SEQ ID NO: 12 and SEQ ID NO: 13, respectively;
the HCDR1-3 and LCDR1-3 described above are determined according to the Chothia numbering scheme.

In some embodiments, the anti-Nectin-4 antibody according to any one of the above comprises a heavy chain variable region and a light chain variable region, wherein:

b. the heavy chain variable region comprises a HCDR1, a HCDR2 and a HCDR3 set forth in SEQ ID NO: 14, SEQ ID NO: 15 and SEQ ID NO: 16, respectively; and the light chain variable region comprises a LCDR1, a LCDR2 and a LCDR3 set forth in SEQ ID NO: 17, SEQ ID NO: 18 and SEQ ID NO: 13, respectively;
the HCDR1-3 and LCDR1-3 described above are determined according to the IMGT numbering scheme.

In some embodiments, the anti-Nectin-4 antibody according to any one of the above comprises a heavy chain variable region and a light chain variable region, wherein:

c. the heavy chain variable region comprises a HCDR1, a HCDR2 and a HCDR3 set forth in SEQ ID NO: 19, SEQ

ID NO: 20 and SEQ ID NO: 10, respectively; and the light chain variable region comprises a LCDR1, a LCDR2 and a LCDR3 set forth in SEQ ID NO: 11, SEQ ID NO: 12 and SEQ ID NO: 13, respectively;
the HCDR1-3 and LCDR1-3 described above are determined according to the Kabat numbering scheme.

The present disclosure provides an anti-Nectin-4 antibody, wherein the anti-Nectin-4 antibody comprises a heavy chain variable region and a light chain variable region, wherein:

a. the heavy chain variable region comprises a HCDR1, a HCDR2 and a HCDR3 set forth in SEQ ID NO: 8, SEQ ID NO: 9 and SEQ ID NO: 10, respectively; and the light chain variable region comprises a LCDR1, a LCDR2 and a LCDR3 set forth in SEQ ID NO: 11, SEQ ID NO: 12 and SEQ ID NO: 13, respectively;
the HCDR1-3 and LCDR1-3 described above are determined according to the Chothia numbering scheme.

The present disclosure provides an anti-Nectin-4 antibody, wherein the anti-Nectin-4 antibody comprises a heavy chain variable region and a light chain variable region, wherein:

b. the heavy chain variable region comprises a HCDR1, a HCDR2 and a HCDR3 set forth in SEQ ID NO: 14, SEQ ID NO: 15 and SEQ ID NO: 16, respectively; and the light chain variable region comprises a LCDR1, a LCDR2 and a LCDR3 set forth in SEQ ID NO: 17, SEQ ID NO: 18 and SEQ ID NO: 13, respectively;
the HCDR1-3 and LCDR1-3 described above are determined according to the IMGT numbering scheme.

The present disclosure provides an anti-Nectin-4 antibody, wherein the anti-Nectin-4 antibody comprises a heavy chain variable region and a light chain variable region, wherein:

c. the heavy chain variable region comprises a HCDR1, a HCDR2 and a HCDR3 set forth in SEQ ID NO: 19, SEQ ID NO: 20 and SEQ ID NO: 10, respectively; and the light chain variable region comprises a LCDR1, a LCDR2 and a LCDR3 set forth in SEQ ID NO: 11, SEQ ID NO: 12 and SEQ ID NO: 13, respectively;
the HCDR1-3 and LCDR1-3 described above are determined according to the Kabat numbering scheme.

In some embodiments, the anti-Nectin-4 antibody according to any one of the above is a human antibody or an antigen-binding fragment thereof.
In some embodiments, the anti-Nectin-4 antibody according to any one of the above comprises a heavy chain variable region and a light chain variable region, wherein:

the heavy chain variable region has at least 90%, 91%, 92%, 93%, 94%, 95%, 96%, 97%, 98%, 99% or 100% sequence identity to SEQ ID NO: 6, and/or the light chain variable region has at least 90%, 91%, 92%, 93%, 94%, 95%, 96%, 97%, 98%, 99% or 100% sequence identity to SEQ ID NO: 7;
in some embodiments, the anti-Nectin-4 antibody according to any one of the above comprises a heavy chain variable region and a light chain variable region, wherein:

the heavy chain variable region is set forth in SEQ ID NO: 6, and the light chain variable region is set forth in SEQ ID NO: 7; or
in some embodiments, the anti-Nectin-4 antibody according to any one of the above further comprises an antibody heavy chain constant region and an antibody light chain constant region; preferably, the heavy chain constant region is selected from the group consisting of human IgG1, IgG2, IgG3 and IgG4 constant regions, and the light chain constant region is selected from the group consisting of human antibody κ and λ chain constant regions; more preferably, the antibody comprises a heavy chain constant region set forth in SEQ ID NO: 4 and a light chain constant region set forth in SEQ ID NO: 5.

In some embodiments, the anti-Nectin-4 antibody according to any one of the above comprises:
a heavy chain having at least 85%, 90%, 91%, 92%, 93%, 94%, 95%, 96%, 97%, 98%, 99% or 100% sequence identity to SEQ ID NO: 21, and/or a light chain having at least 85%, 90%, 91%, 92%, 93%, 94%, 95%, 96%, 97%, 98%, 99% or 100% sequence identity to SEQ ID NO: 22.
In some embodiments, the anti-Nectin-4 antibody according to any one of the above comprises:
a heavy chain set forth in SEQ ID NO: 21 and a light chain set forth in SEQ ID NO: 22.
In some embodiments, the anti-Nectin-4 antibody according to any one of the above binds to Nectin-4 protein expressed by T47D cells or MDA-MB-468 cells with an $EC_{50}$ value of less than 0.1 nM, as determined by FACS.
In some embodiments, the present disclosure further provides an anti-Nectin-4 antibody, wherein the antibody competes for binding to human Nectin-4 with the anti-Nectin-4 antibody according to any one of the above.

In some embodiments, the present disclosure further provides a nucleic acid molecule encoding the anti-Nectin-4 antibody according to any one of the above.

In some embodiments, the present disclosure further provides a host cell comprising the nucleic acid molecule according to any one of the above.

In some embodiments, the present disclosure further provides a pharmaceutical composition comprising a therapeutically effective amount of the anti-Nectin-4 antibody according to any one of the above, or the nucleic acid molecule described above, and one or more pharmaceutically acceptable carriers, diluents or excipients.

In some embodiments, the present disclosure further provides an immunoconjugate comprising the anti-Nectin-4 antibody according to any one of the above and an effector molecule, wherein the effector molecule is coupled to the anti-Nectin-4 antibody; preferably, the effector molecule is selected from the group consisting of a radioisotope, an anti-tumor agent, an immunomodulator, a biological response modifier, a lectin, a cytotoxic drug, a chromophore, a fluorophore, a chemiluminescent compound, an enzyme, a metal ion, and any combination thereof.

In some embodiments, the present disclosure further provides a method for immunodetection or determination of Nectin-4, the method comprising a step of contacting the anti-Nectin-4 antibody according to any one of the above with a subject or a sample from the subject.

In some embodiments, the present disclosure further provides an antibody-drug conjugate of general formula (Pc-L-Y-D) or a pharmaceutically acceptable salt thereof:

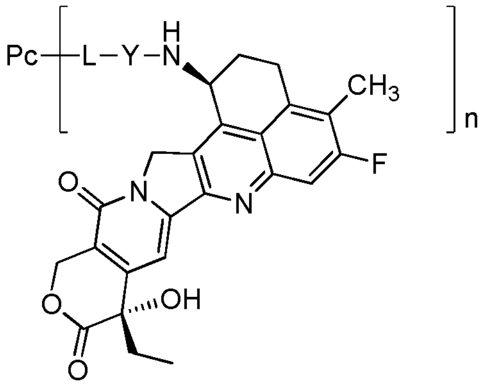

(Pc-L-Y-D)

wherein:

Y is selected from the group consisting of -O-$(CR^aR^b)_m$-$CR^1R^2$-C(O)-, -O-$CR^1R^2$-$(CR^aR^b)_m$-, -O-$CR^1R^2$-, -NH-$(CR^aR^b)_m$-$CR^1R^2$-C(O)- and -S-$(CR^aR^b)_m$-$CR^1R^2$-C(O)-;

$R^a$ and $R^b$ are identical or different and are each independently selected from the group consisting of hydrogen, deuterium, halogen, alkyl, haloalkyl, deuterated alkyl, alkoxy, hydroxy, amino, cyano, nitro, hydroxyalkyl, cycloalkyl and heterocyclyl; or $R^a$ and $R^b$, together with the carbon atom to which they are attached, form cycloalkyl or heterocyclyl;

$R^1$ is selected from the group consisting of halogen, haloalkyl, deuterated alkyl, cycloalkyl, cycloalkylalkyl, alkoxyalkyl, heterocyclyl, aryl and heteroaryl; $R^2$ is selected from the group consisting of hydrogen, halogen, haloalkyl, deuterated alkyl, cycloalkyl, cycloalkylalkyl, alkoxyalkyl, heterocyclyl, aryl and heteroaryl; or $R^1$ and $R^2$, together with the carbon atom to which they are attached, form cycloalkyl or heterocyclyl;

or $R^a$ and $R^2$, together with the carbon atom to which they are attached, form cycloalkyl or heterocyclyl;

m is an integer from 0 to 4; as a non-limiting example, m is selected from the group consisting of 0, 1, 2, 3 and 4;

n is from 1 to 10;

L is a linker unit;

Pc is the anti-Nectin-4 antibody according to any one of the above.

In some embodiments, in the antibody-drug conjugate of general formula (Pc-L-Y-D) or the pharmaceutically acceptable salt thereof according to any one of the above, n is from 1 to 8. In some embodiments, n is from 3 to 8.

In some embodiments, in the antibody-drug conjugate of general formula (Pc-L-Y-D) or the pharmaceutically acceptable salt thereof according to any one of the above,
wherein:

Y is -O-$(CR^aR^b)_m$-$CR^1R^2$-C(O)-;

$R^a$ and $R^b$ are identical or different and are each independently selected from the group consisting of hydrogen, deuterium, halogen and $C_{1-6}$ alkyl;

$R^1$ is $C_{1-6}$ haloalkyl or $C_{3-6}$ cycloalkyl;

$R^2$ is selected from the group consisting of hydrogen, $C_{1-6}$ haloalkyl and $C_{3-6}$ cycloalkyl; or $R^1$ and $R^2$, together with the carbon atom to which they are attached, form $C_{3-6}$ cycloalkyl;

m is 0 or 1.

In some embodiments, in the antibody-drug conjugate of general formula (Pc-L-Y-D) or the pharmaceutically acceptable salt thereof according to any one of the above, Y is selected from the group consisting of:

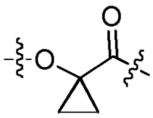, 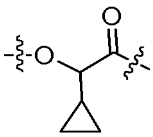, 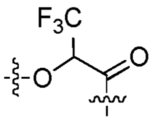, 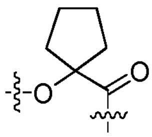, 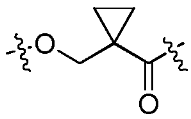, 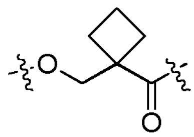

and

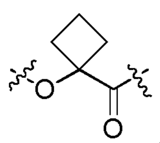;

wherein the O-terminus of Y is connected to the linker unit L.

In some embodiments, in the antibody-drug conjugate of general formula (Pc-L-Y-D) or the pharmaceutically acceptable salt thereof according to any one of the above, the linker unit -L- is $-L^1-L^2-L^3-L^4-$, wherein

$L^1$ is selected from the group consisting of -(succinimidyl-3-yl-*N*)-W-C(O)-, $-CH_2-C(O)-NR^3-W-C(O)-$ and -C(O)-W-C(O)-, wherein W is selected from the group consisting of $C_{1-6}$ alkylene and $C_{1-6}$ alkylene-$C_{3-6}$ cycloalkyl, wherein the $C_{1-6}$ alkylene and $C_{1-6}$ alkylene-$C_{3-6}$ cycloalkyl are each independently optionally further substituted with one or more substituents selected from the group consisting of halogen, hydroxy, cyano, amino, $C_{1-6}$ alkyl, $C_{1-6}$ haloalkyl, deuterated $C_{1-6}$ alkyl, $C_{1-6}$ alkoxy and $C_{3-6}$ cycloalkyl;

$L^2$ is selected from the group consisting of $-NR^4(CH_2CH_2O)p^1CH_2CH_2C(O)-$, $-NR^4(CH_2CH_2O)p^1CH_2C(O)-$, $-S(CH_2)p^1C(O)-$ and a chemical bond, wherein $p^1$ is an integer from 1 to 20;

$L^3$ is a peptide residue consisting of 2 to 7 amino acid residues, wherein the amino acid residues are selected from the group consisting of amino acid residues formed from amino acids from phenylalanine (F), alanine (A), glycine (G), valine (V), lysine (K), citrulline, serine (S), glutamic acid (E) and aspartic acid (D), and are optionally further substituted with one or more substituents selected from the group consisting of halogen, hydroxy, cyano, amino, alkyl, chloroalkyl, deuterated alkyl, alkoxy and cycloalkyl;

$L^4$ is selected from the group consisting of $-NR^5(CR^6R^7)_t-$, $-C(O)NR^5$, $-C(O)NR^5(CH_2)_t-$ and a chemical bond, wherein t is an integer from 1 to 6;

$R^3$, $R^4$ and $R^5$ are identical or different and are each independently selected from the group consisting of hydrogen, $C_{1-6}$ alkyl, $C_{1-6}$ haloalkyl, deuterated $C_{1-6}$ alkyl and $C_{1-6}$ hydroxyalkyl;

$R^6$ and $R^7$ are identical or different and are each independently selected from the group consisting of hydrogen, halogen, $C_{1-6}$ alkyl, $C_{1-6}$ haloalkyl, deuterated $C_{1-6}$ alkyl and $C_{1-6}$ hydroxyalkyl.

In some embodiments, in the antibody-drug conjugate of general formula (Pc-L-Y-D) or the pharmaceutically acceptable salt thereof according to any one of the above, the linker unit -L- is $-L^1-L^2-L^3-L^4-$, wherein

$L^1$ is

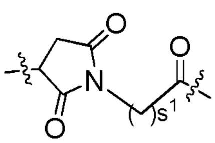,

and $s^1$ is an integer from 2 to 8;

$L^2$ is a chemical bond;

$L^3$ is a tetrapeptide residue;

$L^4$ is $-NR^5(CR^6R^7)_t-$, wherein $R^5$, $R^6$ and $R^7$ are identical or different and are each independently hydrogen or $C_{1-6}$ alkyl, and t is 1 or 2;

the $L^1$ terminus of -L- is connected to Pc, and the $L^4$ terminus is connected to Y

In some embodiments, in the antibody-drug conjugate of general formula (Pc-L-Y-D) or the pharmaceutically acceptable salt thereof according to any one of the above, $L^3$ is a tetrapeptide residue of GGFG (SEQ ID NO: 23).

In some embodiments, in the antibody-drug conjugate of general formula (Pc-L-Y-D) or the pharmaceutically acceptable salt thereof according to any one of the above, -L- is:

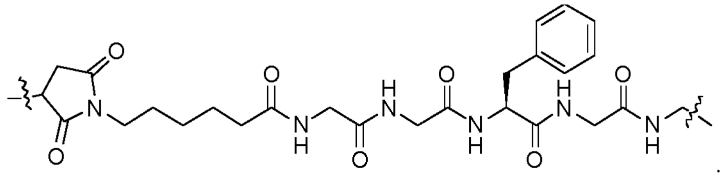

In some embodiments, the antibody-drug conjugate of general formula (Pc-L-Y-D) or the pharmaceutically acceptable salt thereof according to any one of the above is an antibody-drug conjugate of general formula ($Pc-L_a-Y-D$) or a pharmaceutically acceptable salt thereof:

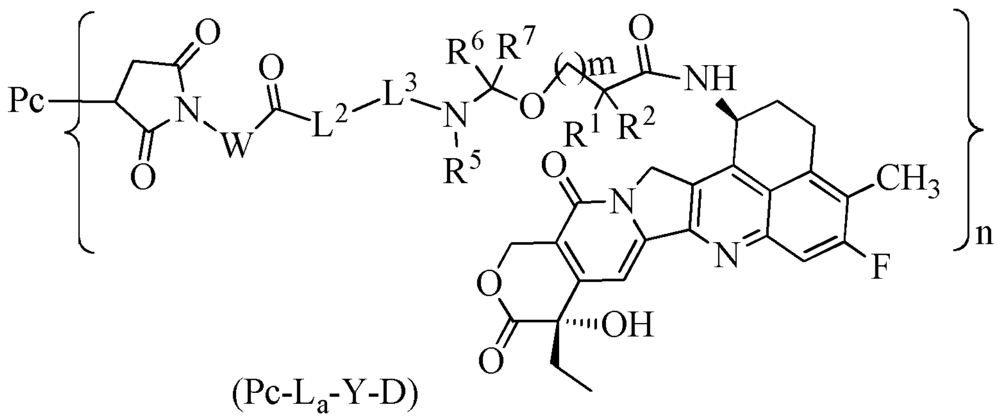

($Pc-L_a-Y-D$)

wherein,

Pc is the anti-Nectin-4 antibody described above;

m is an integer from 0 to 4;

n is from 1 to 10;

$R^1$ is selected from the group consisting of halogen, haloalkyl, deuterated alkyl, cycloalkyl, cycloalkylalkyl, alkoxyalkyl, heterocyclyl, aryl and heteroaryl; $R^2$ is selected from the group consisting of hydrogen, halogen, haloalkyl, deuterated alkyl, cycloalkyl, cycloalkylalkyl, alkoxyalkyl, heterocyclyl, aryl and heteroaryl; or $R^1$ and $R^2$, together with the carbon atom to which they are attached, form cycloalkyl or heterocyclyl;

W is selected from the group consisting of $C_{1-6}$ alkylene and $C_{1-6}$ alkylene-$C_{3-6}$ cycloalkyl, wherein the $C_{1-6}$ alkylene and $C_{3-6}$ cycloalkyl are each independently optionally further substituted with one or more substituents selected from the group consisting of halogen, hydroxy, cyano, amino, $C_{1-6}$ alkyl, $C_{1-6}$ chloroalkyl, deuterated $C_{1-6}$ alkyl, $C_{1-6}$ alkoxy and $C_{3-6}$ cycloalkyl;

$L^2$ is selected from the group consisting of $-NR^4(CH_2CH_2O)p^1CH_2CH_2C(O)-$, $-NR^4(CH_2CH_2O)p^1CH_2C(O)-$, $-S(CH_2)p^1C(O)-$ and a chemical bond, wherein $p^1$ is an integer from 1 to 20;

$L^3$ is a peptide residue consisting of 2 to 7 amino acid residues, wherein the amino acid residues are selected from the group consisting of amino acid residues formed from amino acids from phenylalanine (F), alanine (A), glycine (G), valine (V), lysine (K), citrulline, serine (S), glutamic acid (E) and aspartic acid (D), and are optionally further substituted with one or more substituents selected from the group consisting of halogen, hydroxy, cyano, amino, alkyl, chloroalkyl, deuterated alkyl, alkoxy and cycloalkyl;

$R^5$ is selected from the group consisting of hydrogen, alkyl, haloalkyl, deuterated alkyl and hydroxyalkyl;
$R^6$ and $R^7$ are identical or different and are each independently selected from the group consisting of hydrogen, halogen, alkyl, haloalkyl, deuterated alkyl and hydroxyalkyl.

In some embodiments, the antibody-drug conjugate of general formula (Pc-L-Y-D) or the pharmaceutically acceptable salt thereof according to any one of the above is an antibody-drug conjugate of general formula (Pc-$L_a$-Y-D) or a pharmaceutically acceptable salt thereof, wherein

Pc is the anti-Nectin-4 antibody according to any one of the above;
m is an integer from 0 to 4;
n is from 1 to 10;
$R^1$ is selected from the group consisting of halogen, $C_{1-6}$ haloalkyl, deuterated $C_{1-6}$ alkyl, $C_{3-6}$ cycloalkyl, $C_{3-6}$ cycloalkyl $C_{1-6}$ alkyl, $C_{1-6}$ alkoxy $C_{1-6}$ alkyl, heterocyclyl, aryl and heteroaryl; $R^2$ is selected from the group consisting of hydrogen, halogen, $C_{1-6}$ haloalkyl, deuterated $C_{1-6}$ alkyl, $C_{3-6}$ cycloalkyl, $C_{3-6}$ cycloalkyl $C_{1-6}$ alkyl, $C_{1-6}$ alkoxy $C_{1-6}$ alkyl, heterocyclyl, aryl and heteroaryl; or $R^1$ and $R^2$, together with the carbon atom to which they are attached, form $C_{3-6}$ cycloalkyl or heterocyclyl;
W is selected from the group consisting of $C_{1-6}$ alkylene and $C_{1-6}$ alkylene-$C_{3-6}$ cycloalkyl, wherein the $C_{1-6}$ alkylene and $C_{1-6}$ alkylene-$C_{3-6}$ cycloalkyl are each independently optionally further substituted with one or more substituents selected from the group consisting of halogen, hydroxy, cyano, amino, $C_{1-6}$ alkyl, $C_{1-6}$ chloroalkyl, deuterated $C_{1-6}$ alkyl, $C_{1-6}$ alkoxy and $C_{3-6}$ cycloalkyl;
$L^2$ is selected from the group consisting of $-NR^4(CH_2CH_2O)p^1CH_2CH_2C(O)-$, $-NR^4(CH_2CH_2O)p^1CH_2C(O)-$, $-S(CH_2)p^1C(O)-$ and a chemical bond, wherein $p^1$ is an integer from 1 to 20;
$L^3$ is a peptide residue consisting of 2 to 7 amino acid residues, wherein the amino acid residues are selected from the group consisting of amino acid residues formed from amino acids from phenylalanine (F), alanine (A), glycine (G), valine (V), lysine (K), citrulline, serine (S), glutamic acid (E) and aspartic acid (D), and are optionally further substituted with one or more substituents selected from the group consisting of halogen, hydroxy, cyano, amino, $C_{1-6}$ alkyl, $C_{1-6}$ chloroalkyl, deuterated $C_{1-6}$ alkyl, $C_{1-6}$ alkoxy and $C_{3-6}$ cycloalkyl;
$R^5$ is selected from the group consisting of hydrogen, $C_{1-6}$ alkyl, $C_{1-6}$ haloalkyl, deuterated $C_{1-6}$ alkyl and $C_{1-6}$ hydroxyalkyl;
$R^6$ and $R^7$ are identical or different and are each independently selected from the group consisting of hydrogen, halogen, $C_{1-6}$ alkyl, $C_{1-6}$ haloalkyl, deuterated $C_{1-6}$ alkyl and $C_{1-6}$ hydroxyalkyl;
the heterocyclyl comprises 3 to 6 ring atoms, of which 1 to 3 are heteroatoms selected from the group consisting of nitrogen, oxygen and sulfur.

In some embodiments, in the antibody-drug conjugate of general formula (Pc-L-Y-D) or the pharmaceutically acceptable salt thereof according to any one of the above, the antibody-drug conjugate is:

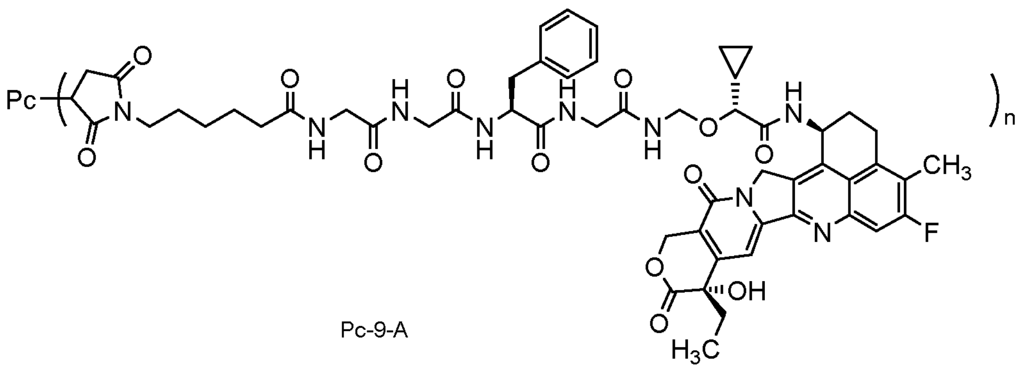

Pc-9-A

wherein:

n is from 1 to 8;
Pc is an anti-Nectin-4 antibody comprising a heavy chain set forth in SEQ ID NO: 21 and a light chain set forth in SEQ ID NO: 22.

In some embodiments, in the antibody-drug conjugate of general formula (Pc-L-Y-D) or the pharmaceutically acceptable salt thereof according to any one of the above, n is from 1 to 8.

In some embodiments, in the antibody-drug conjugate of general formula (Pc-L-Y-D) or the pharmaceutically acceptable salt thereof according to any one of the above, a non-limiting example of m is selected from the group consisting of 0, 1, 2, 3 and 4.

In some embodiments, the present disclosure further provides a method for preparing the antibody-drug conjugate of general formula (Pc-$L_a$-Y-D) or the pharmaceutically acceptable salt thereof according to any one of the above, comprising the following step:

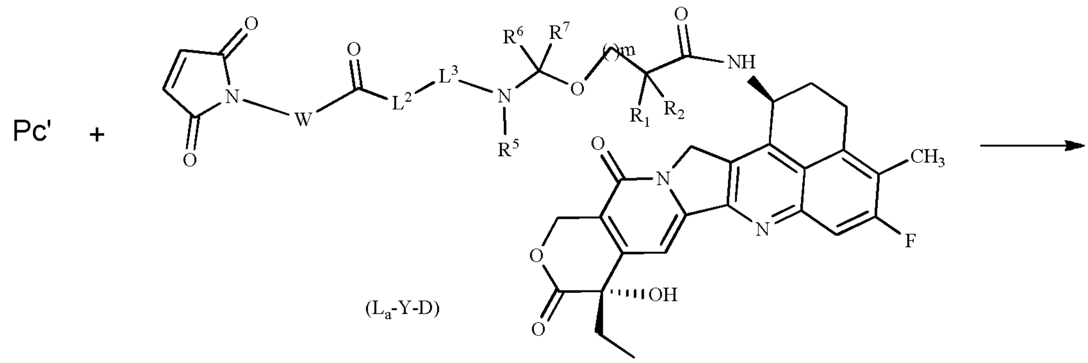

($L_a$-Y-D)

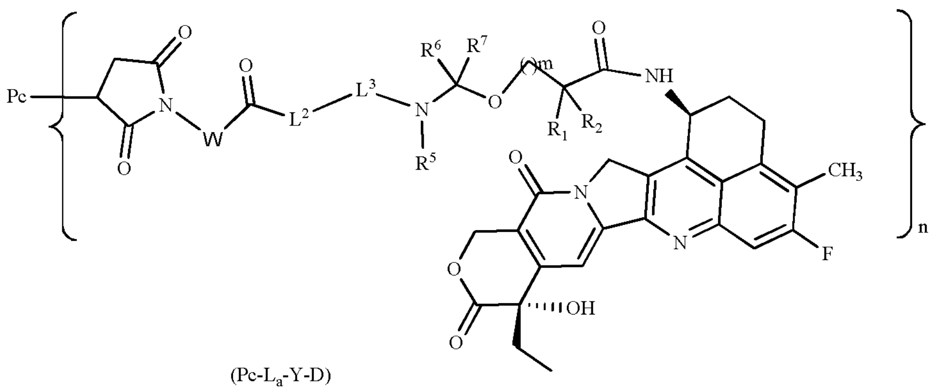

(Pc-$L_a$-Y-D)

conducting a coupling reaction of Pc' with a compound of general formula ($L_a$-Y-D) to obtain a compound of general formula (Pc-$L_a$-Y-D);

wherein:

Pc' is obtained by reducing Pc;

n, m, W, $L^2$, $L^3$, $R^1$, $R^2$, $R^5$, $R^6$ and $R^7$ are as defined in any one of the above.

In some embodiments, n is an average value from 0-10, preferably 1-10, more preferably 1-8, or 2-8, or 2-7, or 2-4, or 3-8, or 3-7, or 3-6, or 4-7, or 4-6, or 4-5; in some embodiments, n is an average value from 1, 2, 3, 4, 5, 6, 7, 8, 9 and 10.

In some embodiments, the present disclosure further provides a pharmaceutical composition comprising the anti-Nectin-4 antibody according to any one of the above, or the nucleic acid molecule according to any one of the above, or the antibody-drug conjugate or the pharmaceutically acceptable salt thereof according to any one of the above, and one or more pharmaceutically acceptable excipients, diluents or carriers.

In some embodiments, the present disclosure further provides use of the anti-Nectin-4 antibody according to any one of the above, or the nucleic acid molecule according to any one of the above, or the antibody-drug conjugate or the pharmaceutically acceptable salt thereof according to any one of the above, or the pharmaceutical composition according to any one of the above, in the preparation of a medicament for treating a Nectin-4-mediated disease or disorder.

In some embodiments, the present disclosure further provides use of the anti-Nectin-4 antibody according to any one of the above, or the nucleic acid molecule according to any one of the above, or the antibody-drug conjugate or the pharmaceutically acceptable salt thereof according to any one of the above, or the pharmaceutical composition according to any one of the above, in the preparation of a medicament for treating and/or preventing tumors and cancers, wherein the tumors and cancers are selected from the group consisting of breast cancer, pancreatic cancer, lung cancer, esophageal cancer, non-small cell lung cancer, laryngeal tumors, pharyngeal tumors, oral tumors, gastric cancer, ovarian

cancer, prostate cancer, bladder cancer, colorectal cancer, head and neck cancer, squamous cell carcinoma and melanoma.

In some embodiments, the present disclosure further provides a kit comprising the anti-Nectin-4 antibody according to any one of the above, or the nucleic acid molecule according to any one of the above, or the antibody-drug conjugate or the pharmaceutically acceptable salt thereof according to any one of the above, or the pharmaceutical composition according to any one of the above.

In some embodiments, the present disclosure further provides a method for preventing or treating a disease or disorder, the method comprising administering to a subject a therapeutically effective amount of the anti-Nectin-4 antibody according to any one of the above, or the nucleic acid molecule according to any one of the above, or the antibody-drug conjugate or the pharmaceutically acceptable salt thereof according to any one of the above, or the pharmaceutical composition according to any one of the above. In some embodiments, the disease or disorder is preferably a tumor, an autoimmune disease, or an infectious disease; in some embodiments, the disease or disorder is a disease or disorder associated with Nectin-4.

In another aspect, the present disclosure provides a pharmaceutical composition comprising the anti-Nectin-4 antibody, the antibody-drug conjugate or the pharmaceutically acceptable salt thereof according to any one of the above, and one or more pharmaceutically acceptable excipients, diluents or carriers. In some embodiments, a unit dose of the pharmaceutical composition comprises 0.1 mg-3000 mg or 1 mg-1000 mg of the anti-Nectin-4 antibody described above or the antibody-drug conjugate described above.

In another aspect, the present disclosure provides use of the antibody-drug conjugate or the pharmaceutically acceptable salt thereof or the pharmaceutical composition comprising same according to any one of the above as a medicament.

In another aspect, the present disclosure provides use of the antibody-drug conjugate or the pharmaceutically acceptable salt thereof or the pharmaceutical composition comprising same according to any one of the above in the preparation of a medicament for treating a Nectin-4-mediated disease or disorder; in some embodiments, the Nectin-4-mediated disease or disorder is a cancer with high, moderate or low Nectin-4 expression.

In another aspect, the present disclosure provides use of the antibody-drug conjugate or the pharmaceutically acceptable salt thereof or the pharmaceutical composition comprising same according to any one of the above in the preparation of a medicament for treating or preventing a tumor or cancer; in some embodiments, the tumor or cancer is selected from the group consisting of breast cancer, pancreatic cancer, lung cancer, esophageal cancer, non-small cell lung cancer, laryngeal tumors, pharyngeal tumors, oral tumors, gastric cancer, ovarian cancer, prostate cancer, bladder cancer, colorectal cancer, head and neck cancer, squamous cell carcinoma and melanoma.

In another aspect, the present disclosure further relates to a method for treating and/or preventing a tumor, the method comprising administering to a subject in need thereof a therapeutically effective dose of the antibody-drug conjugate or the pharmaceutically acceptable salt thereof or the pharmaceutical composition comprising same according to any one of the above; in some embodiments, the tumor is a cancer associated with high Nectin-4 expression, a cancer associated with moderate Nectin-4 expression, or a cancer associated with low Nectin-4 expression.

In another aspect, the present disclosure further relates to a method for treating or preventing a tumor or cancer, the method comprising administering to a subject in need thereof a therapeutically effective dose of the antibody-drug conjugate or the pharmaceutically acceptable salt thereof or the pharmaceutical composition comprising same according to any one of the above; in some embodiments, the tumor or cancer is selected from the group consisting of breast cancer, pancreatic cancer, lung cancer, esophageal cancer, non-small cell lung cancer, laryngeal tumors, pharyngeal tumors, oral tumors, gastric cancer, ovarian cancer, prostate cancer, bladder cancer, colorectal cancer, head and neck cancer, squamous cell carcinoma and melanoma.

In another aspect, the present disclosure further provides the anti-Nectin-4 antibody or the antibody-drug conjugate thereof according to any one of the above as a medicament, and in some embodiments, as a medicament for treating a cancer or tumor, more preferably as a medicament for treating a Nectin-4-mediated cancer.

The active compound may be made into a form suitable for administration by any suitable route. The active compound may be in the form of a unit dose, or in the form of a single dose that subjects can administer to themselves. The unit dose of the compound or composition of the present disclosure may be expressed in the form of a tablet, a capsule, a cachet, a vial, a powder, a granule, a lozenge, a suppository, a regenerating powder, or a liquid formulation.

The dose of the compound or composition used in the treatment method of the present disclosure generally varies depending on the severity of the disease, the weight of the subject, and the relative efficacy of the compound. However, as a general instruction, a suitable unit dose may be 0.1 mg-1000 mg.

The pharmaceutical composition of the present disclosure may comprise, in addition to the active compound, one or more excipients selected from the group consisting of a filler, a diluent, a binder, a wetting agent, a disintegrant, an excipient and the like. Depending on the method of administration, the composition may comprise 0.1 wt.% to 99 wt.% of the active compound.

The Nectin-4 antibody provided by the present disclosure has a good affinity for a cell surface antigen and

good endocytosis efficiency; the Nectin-4 antibody-drug conjugate provided by the present disclosure has very high tumor-inhibiting efficiency, and has better efficacy and lower toxic and side effects in animals.

## BRIEF DESCRIPTION OF THE DRAWINGS

FIG. 1 shows the binding of the antibody of the present disclosure to Nectin4-CHOK1 cells.
FIG. 2 shows the binding of the antibody of the present disclosure to T47D cells.
FIG. 3 shows the binding of the antibody of the present disclosure to MDA-MB-468 cells.
FIG. 4 shows the endocytic activity of the antibody of the present disclosure in a DT3C test.
FIG. 5A shows the endocytic activity of the antibody of the present disclosure (20 nM) in Nectin4-CHOK1 cells in a pHrodo test.
FIG. 5B shows the endocytic activity of the antibody of the present disclosure (5 nM) in Nectin4-CHOK1 cells in a pHrodo test.
FIG. 6 shows the efficacy of the ADC samples of the present disclosure on xenograft tumors in T47D tumor-bearing mice.
FIG. 7 shows the pharmacokinetics of the ADC sample of the present disclosure in Nectin4 F344 RG rats.

## Detailed Description of the Invention

### Terms

Unless otherwise defined, all technical and scientific terms used herein have the same meaning as commonly understood by those of ordinary skill in the art to which the present disclosure belongs. Although any methods and materials similar or equivalent to those described herein can also be used to implement or test the present disclosure, preferred methods and materials are described herein. In describing and claiming the present disclosure, the following terms are used in accordance with the definitions below.

When a trade name is used in the present disclosure, it is intended to include the formulation of the commercial product under the trade name, and the drug and active drug component of the commercial product under the trade name.

Unless otherwise stated, the terms used in the specification and claims have the following meanings.

The three-letter and single-letter codes for amino acids used herein are as described in J. biol. chem, 243, p3558 (1968).

The term "amino acid" refers to naturally occurring and synthetic amino acids, as well as amino acid analogs and amino acid mimics that function in a manner similar to the naturally occurring amino acids. Naturally occurring amino acids are those encoded by genetic codes and those amino acids later modified, e.g., hydroxyproline, $\gamma$-carboxyglutamic acid, and O-phosphoserine. Amino acid analogs refer to compounds that have a substantially identical chemical structure (i.e., an $\alpha$ carbon that binds to hydrogen, carboxyl, amino, and an R group) to naturally occurring amino acids, e.g., homoserine, norleucine, methionine sulfoxide, and methioninemethyl sulfonium. Such analogs have a modified R group (e.g., norleucine) or a modified peptide skeleton, but retain a substantially identical chemical structure to naturally occurring amino acids. Amino acid mimics refer to chemical compounds that have a structure different from the general chemical structure of amino acids, but function in a manner similar to naturally occurring amino acids.

The term "antibody" is used herein in the broadest sense and encompasses a variety of antibody structures, including but not limited to monoclonal antibodies, polyclonal antibodies, multispecific antibodies (e.g., bispecific antibodies), and full-length antibodies, and antibody fragments (or antigen-binding fragments, or antigen-binding moieties) so long as they exhibit the desired antigen-binding activity.

"Native antibody" refers to a naturally-occurring immunoglobulin molecule. For example, a native IgG antibody is a heterotetrameric glycoprotein of about 150,000 Daltons composed of two identical light chains and two identical heavy chains linked by a disulfide bond. From N-terminus to C-terminus, each heavy chain has one variable region (VH), also known as variable heavy domain or heavy chain variable domain, followed by three constant domains (CH1, CH2, and CH3). Similarly, from the N-terminus to the C-terminus, each light chain has one variable region (VL), also known as variable light domain or light chain variable domain, followed by one constant light domain (also known as a light chain constant region, CL).

The term "full-length antibody", "intact antibody", and "whole antibody" are used herein interchangeably and refer to an antibody comprising a substantially similar structure to a native antibody structure or whose heavy chains have an Fc region as defined herein.

An "isolated" antibody is one that has been separated from components of its natural environment. In the present disclosure, in some embodiments, the antibody may be purified to a purity of greater than 90% or a purity of

99%. In some embodiments, the antibody is purified and assayed by methods such as electrophoresis (e.g., SDS-PAGE, isoelectric focusing (IEF), capillary electrophoresis) or chromatography (e.g., ion exchange or reversed-phase HPLC).

The term "variable region" or "variable domain" refers to a domain in antibody heavy and/or light chains that is involved in the binding of the antibody to an antigen. The VH and VL of a native IgG antibody each comprise four conserved framework regions (FRs) and three complementarity determining regions (CDRs). The term "complementarity determining region" or "CDR" refers to a region in the variable domain that primarily contributes to antigen binding; "framework" or "FR" refers to variable domain residues other than CDR residues in variable domains. A VH comprises 3 CDR regions: HCDR1, HCDR2, and HCDR3; a VL comprises 3 CDR regions: LCDR1, LCDR2, and LCDR3. Each VH and VL is composed of three CDRs and four FRs arranged from the amino terminus to the carboxyl terminus in the following order: FR1, CDR1, FR2, CDR2, FR3, CDR3, and FR4. A single VH or VL may be sufficient to provide antigen-binding specificity.

The amino acid sequence boundaries of the CDRs can be determined by a variety of well-known schemes, for example, the "Kabat" numbering scheme (see Kabat et al. (1991), "Sequences of Proteins of Immunological Interest", 5th ed., Public Health Service, National Institutes of Health, Bethesda, MD), the "Chothia" numbering scheme, the "ABM" numbering scheme, the "contact" numbering scheme (see Martin, ACR. Protein Sequence and Structure Analysis of Antibody Variable Domains[J]. 2001), and the ImMunoGenTics (IMGT) numbering scheme (Lefranc, M.P. et al., Dev. Comp. Immunol., 27, 55-77 (2003); Front Immunol. 2018 Oct 16; 9: 2278), and the like. The corresponding relationships among the various numbering schemes are well known to those skilled in the art and are exemplary, as shown in Table 1 below.

Table 1. Relationships among CDR numbering schemes

| CDR | IMGT | Kabat | AbM | Chothia | Contact |
|---|---|---|---|---|---|
| HCDR1 | 27-38 | 31-35 | 26-35 | 26-32 | 30-35 |
| HCDR2 | 56-65 | 50-65 | 50-58 | 52-56 | 47-58 |
| HCDR3 | 105-117 | 95-102 | 95-102 | 95-102 | 93-101 |
| LCDR1 | 27-38 | 24-34 | 24-34 | 24-34 | 30-36 |
| LCDR2 | 56-65 | 50-56 | 50-56 | 50-56 | 46-55 |
| LCDR3 | 105-117 | 89-97 | 89-97 | 89-97 | 89-96 |

The term "light chain" includes the variable region domain VL and the constant region domain CL. VL is at the amino terminus of a light chain. Light chains include κ and λ chains.

The term "heavy chain" includes the variable region domain VH and the three constant region domains CH1, CH2 and CH3. VH is at the amino terminus of a heavy chain and the CH domains are at the carboxy terminus, with CH3 being closest to the carboxy terminus of a polypeptide. A heavy chain may be of any isotype, including IgG (including IgG1, IgG2, IgG3 and IgG4 subtypes), IgA (including IgA1 and IgA2 subtypes), IgM and IgE.

The term "antibody fragment" refers to a molecule different from an intact antibody, which comprises a moiety of an intact antibody that binds to an antigen to which the intact antibody binds. Antibody fragments include, but are not limited to, Fv, Fab, Fab', Fab'-SH, $F(ab')_2$, single-domain antibodies, single-chain Fab (scFab), diabodies, linear antibodies, single-chain antibody molecules (e.g., scFv), and multispecific antibodies formed from antibody fragments.

The term "Fc region" or "fragment crystallizable region" is used to define the C-terminal region of the heavy chain of an antibody, including native sequence Fc regions and modified Fc regions. In some embodiments, the Fc region of the human IgG heavy chain is defined as extending from the amino acid residue at position Cys226 or from Pro230 to its carboxyl terminus. The boundaries of the Fc region of the heavy chain of an antibody may also be varied, for example, by deleting the C-terminal lysine of the Fc region (residue 447 according to the EU numbering scheme) or deleting the C-terminal glycine and lysine of the Fc region (residues 446 and 447 according to the EU numbering scheme). Thus, in some embodiments, a composition of intact antibodies may comprise antibody populations with all K447 residues and/or G446 + K447 residues removed. In some embodiments, a composition of intact antibodies may comprise antibody populations without K447 residue and/or G446 + K447 residue removed. In some embodiments, a composition of intact antibodies comprises antibody populations having a mixture of antibodies with and without K447 residues and/or G446 + K447 residues. Suitable native sequence Fc regions for the antibodies described herein include human IgG1, IgG2 (IgG2A, IgG2B), IgG3, and IgG4. Unless otherwise specified herein, the numbering of amino acid residues in the Fc region or constant region conforms to the EU numbering scheme, also known as the EU index, as described in Kabat et al., Sequences of Proteins of Immunological Interest, 5th edition, Public Health Service, National Institutes of Health, Bethesda, MD, 1991.

The term "chimeric" antibody refers to an antibody in which a portion of the heavy and/or light chains in the antibody is derived from a particular source or species, while the remainder of the heavy and/or light chains is derived from a different source or species.

The term "humanized" antibody refers to an antibody that retains the reactivity of a non-human antibody while having low immunogenicity in humans. For example, this can be achieved by retaining the non-human CDR regions and replacing the remainder of the antibody with its human counterparts (i.e., the constant regions and the framework region portion of the variable regions).

The term "human antibody" refers to an antibody in which the variable and constant regions are human sequences. The term encompasses antibodies that are derived from human genes but have, for example, sequences that have been altered to, e.g., reduce possible immunogenicity, increase affinity, eliminate cysteines that may cause undesired folding, or generate glycosylation sites. The term encompasses antibodies recombinantly produced in non-human cells that may confer glycosylation not characteristic of human cells. The term also encompasses antibodies that have been cultured in transgenic mice comprising some or all of the human immunoglobulin heavy and light chain loci. The meaning of the human antibody specifically excludes humanized antibodies comprising non-human antigen-binding residues.

The term "affinity-matured" antibody refers to an antibody with one or more alterations in one or more CDRs thereof which result in an improvement in the affinity of the antibody for antigens, compared to the parent antibody. In some embodiments, an affinity-matured antibody has nanomolar or even picomolar affinity for the target antigen. Affinity-matured antibodies can be produced using methods known in the art. Marks et al., Bio/Technology 10:779-783 (1992) describes affinity maturation by VH and VL domain shuffling. The following documents describe random mutagenesis of CDR and/or framework residues: Barbas et al., PNAS, 91:3809-3813 (1994); Schier et al., Gene 169:147-155 (1995); Yelton et al., J.Immunol. 155:1994-2004 (1995); Jackson et al., J.Immunol. 154 (7):3310-9 (1995) and Hawkins et al., J. Mol. Biol. 226:889-896 (1992).

The term "monoclonal antibody" refers to a population of substantially homogeneous antibodies, that is, the amino acid sequences of the antibody molecules comprised in the population are identical, except for a small number of natural mutations that may exist. In contrast, polyclonal antibody preparations generally comprise a plurality of different antibodies having different amino acid sequences in their variable domains, which are generally specific for different epitopes. "Monoclonal" refers to the characteristics of an antibody obtained from a substantially homogeneous population of antibodies, and should not be construed as requiring the production of the antibody by any particular method. For example, monoclonal antibodies can be prepared by the hybridoma method described in Kohler et al., (1975) Nature 256:495, or by recombinant DNA methods (see, e.g., U.S. Pat. No. 4,816,567). Or "monoclonal antibodies" can be isolated from phage antibody libraries using the techniques described in Clackson et al., (1991) Nature 352: 624-628 and Marks et al., (1991) J. Mol. Biol. 222:581-597. Or see Presta (2005) J. Allergy Clin. Immunol. 116:731, or "monoclonal antibodies" can be prepared using methods utilizing transgenic animals containing all or part of the human immunoglobulin loci.

The term "antigen" refers to a molecule or a molecular moiety that is capable of being selectively bound by an antibody and is capable of being used in an animal to produce an antibody that is capable of binding to the antigen. An antigen may have one or more epitopes capable of interacting with different antibodies.

The term "epitope" refers to an area or region on an antigen that is capable of specifically binding to an antibody. Epitopes can be formed from contiguous strings of amino acids (linear epitope) or comprise non-contiguous amino acids (conformational epitope), e.g. coming in spatial proximity due to the folding of the antigen (i.e. by the tertiary folding of a proteinaceous antigen). The difference between the conformational epitope and the linear epitope is that in the presence of denaturing solvents, the binding of the antibody to the conformational epitope is lost. An epitope comprises at least 3, at least 4, at least 5, at least 6, at least 7, or 8-10 amino acids in a unique spatial conformation.

Screening for antibodies that bind to particular epitopes (i.e., those that bind to identical epitopes) can be performed using methods well known in the art, including but not limited to alanine scanning, peptide blotting (see Meth. Mol. Biol. 248 (2004) 443-463), peptide cleavage analysis, epitope excision, epitope extraction, chemical modification of the antigen (see Prot. Sci. 9 (2000) 487-496), and cross-blocking (see "Antibodies", Harlow and Lane (Cold Spring Harbor Press, Cold Spring Harb., NY)).

"An antibody that binds to the same epitope" as a reference antibody or "an antibody that competes for binding with" a reference antibody refers to an antibody that blocks binding of the reference antibody to an antigen by 50% or more, or an antibody whose binding to an antigen is blocked by 50% or more by the reference antibody, in a competition assay. For example, to determine whether the test antibody binds to the same epitope as the reference antibody, the reference antibody is allowed to bind to the antigen under saturating conditions. After removal of excess reference antibody, the ability of the test antibody to bind to the antigen is assessed. To confirm whether the test antibody binds to the same epitope or is just hampered from binding by steric reasons, conventional experimentation can be used (e.g., peptide mutation and binding analyses using ELISA, RIA, surface plasmon resonance, flow cytometry or any other quantitative or qualitative antibody-binding assay available in the art). This assay should be carried out in two set-ups,

i.e. with both of the antibodies being saturating antibodies. If, in both set-ups, only the first (saturating) antibody is capable of binding to the antigen, then it can be concluded that the test antibody and the reference antibody compete for binding to the antigen.

In some embodiments, two antibodies are considered to bind to the same epitope or an overlapping epitope if a 1-, 5-, 10-, 20- or 100-fold excess of one antibody inhibits binding of the other by at least 50%, at least 75%, at least 90% or even 99% or more as measured in a competitive binding assay (see, e.g., Junghans et al., Cancer Res. 50 (1990) 1495-1502).

In some embodiments, two antibodies are considered to bind to the same epitope if essentially all amino acid mutations in the antigen that reduce or eliminate binding of one antibody reduce or eliminate binding of the other. Two antibodies are considered to have "overlapping epitopes" if only some of the mutations reduce or eliminate binding of the other.

The term "affinity" refers to the overall strength of a non-covalent interaction between a single binding site of a molecule (e.g., an antibody) and its binding partner (e.g., an antigen). Unless otherwise indicated, as used herein, binding "affinity" refers to an internal binding affinity that reflects a 1:1 interaction between members of a binding pair (e.g., an antibody and an antigen). The affinity of a molecule X for its ligand Y can be generally expressed by the equilibrium dissociation constant (KD). Affinity can be determined by conventional methods known in the art, including those described herein. As used herein, the term "kassoc" or "ka" refers to the association rate of a particular antibody-antigen interaction, while the term "kdis" or "kd" refers to the dissociation rate of a particular antibody-antigen interaction. The term "KD" refers to the equilibrium dissociation constant, which is obtained from the ratio of kd to ka (i.e., kd/ka) and denoted by a molar concentration (M). The KD value of an antibody can be determined using methods well known in the art. For example, surface plasmon resonance is determined using a biosensing system such as a system, or affinity in a solution is determined by solution equilibrium titration (SET).

The term "specifically bind", "specific binding" or "binds" refers to antibody binding to an antigen or an epitope within the antigen with greater affinity than to other antigens or epitopes. Typically, the antibody binds to the antigen or the epitope within the antigen with an equilibrium dissociation constant (KD) of about $1 \times 10^{-7}$ M or less (e.g., about $1 \times 10^{-8}$ M or less, about $1 \times 10^{-9}$ M or less). In some embodiments, the KD for the binding of an antibody to an antigen is 10% or less (e.g., 1%) of the KD for the binding of the antibody to a non-specific antigen (e.g., BSA or casein). KD may be determined using known methods in the art, for example, by a BIACORE' surface plasmon resonance assay. However, an antibody that specifically binds to an antigen or an epitope within the antigen may have cross-reactivity to other related antigens, e.g. to corresponding antigens from other species (homologous), such as humans or monkeys, e.g., Macaca fascicularis (cynomolgus, cyno), Pan troglodytes (chimpanzee, chimp), or Callithrix jacchus (commonmarmoset, marmoset).

The terms "anti-Nectin-4 antibody" and "antibody that binds to Nectin-4" refer to an antibody that is capable of binding to Nectin-4 with sufficient affinity. In certain embodiments, the antibody that binds to an anti-Nectin-4 antibody has an equilibrium dissociation constant (KD) of < about 1 μM, < about 100 nM, < about 10 nM, < about 1 nM, < about 0.1 nM, < about 0.01 nM, or < about 0.001 nM (e.g., $10^{-8}$ M or less, e.g., $10^{-8}$ M to $10^{-9}$ M, e.g., $10^{-9}$ M or less). In certain embodiments, the anti-Nectin-4 antibody binds to an epitope of conserved Nectin-4 among Nectin-4 from different species.

The term "antibody-dependent cell-mediated cytotoxicity" or "ADCC" refers to a form of cytotoxicity in which secreted Ig bound onto Fc receptors (FcRs) present on certain cytotoxic cells (e.g., natural killer (NK) cells, neutrophils and macrophages) enables these cytotoxic effector cells to bind specifically to antigen-bearing target cells and subsequently kill the target cells with cytotoxins. The antibodies "arm" the cytotoxic cells and are essential for such killing. The primary cells for mediating ADCC, NK cells, express FcγRIII only, whereas monocytes express FcγRI, FcγRII and FcγRIII. FcR expression on hematopoietic cells is summarized in Table 3 on page 464 of Ravetch and Kinet, Annu. Rev. Immunol, 9:457-92 (1991). To assess the ADCC activity of a target molecule, an *in vitro* ADCC assay, such as that described in U.S. Pat. No. 5,500,362 or 5,821,337 may be performed. Useful effector cells for such assays include peripheral blood mononuclear cells (PBMCs) and natural killer (NK) cells. Alternatively, the ADCC activity of the target molecule may be assessed *in vivo,* e.g., in an animal model (such as that disclosed in Clynes et al. (USA) 95:652-656 (1998)).

The term "antibody-dependent cellular phagocytosis" ("ADCP") refers to a mechanism by which antibody-coated target cells are eliminated by internalization of phagocytic cells such as macrophages or dendritic cells.

The term "complement-dependent cytotoxicity" or "CDC" refers to a mechanism for inducing cell death in which the Fc effector domain of a target-binding antibody binds to and activates the complement component C1q, and C1q then activates the complement cascade, resulting in the death of the target cell. Activation of a complement may also result in the deposition of complement components on the surface of target cells that promote CDC by binding to complement receptors on leukocytes (e.g., CR3).

The term "nucleic acid" is used interchangeably herein with the term "polynucleotide" and refers to deoxyribonucleotide or ribonucleotide and a polymer thereof in either single-stranded or double-stranded form. The term encompasses nucleic acids comprising known nucleotide analogs or modified backbone residues or linkages, which are synthetic, naturally occurring, and non-naturally occurring, have similar binding properties to the reference nucleic acid, and are metabolized in a manner similar to the reference nucleotide. Examples of such analogs include, but are not limited to, phosphorothioate, phosphoramidate, methylphosphonate, chiral-methylphosphonate, 2-O-methyl ribonucleotide, and peptide-nucleic acid (PNA). "Isolated" nucleic acid refers to a nucleic acid molecule that has been separated from components of its natural environment. The isolated nucleic acid includes a nucleic acid molecule comprised in a cell that generally comprises the nucleic acid molecule, but the nucleic acid molecule is present extrachromosomally or at a chromosomal position different from its natural chromosomal position. "Nucleic acid encoding the anti-Nectin-4 antibody" refers to one or more nucleic acid molecules encoding the antibody heavy and light chains (or fragments thereof).

Unless otherwise stated, a particular nucleic acid sequence also implicitly encompasses conservatively modified variants thereof (e.g., degenerate codon substitutions) and complementary sequences, as well as the sequence explicitly indicated. Specifically, as detailed below, degenerate codon substitutions may be obtained by generating sequences in which the third position of one or more selected (or all) codons is substituted with mixed bases and/or deoxyinosine residues (Batzer et al., Nucleic Acid Res. 19:5081, 1991; Ohtsuka et al., J. Biol. Chem. 260:2605-2608, 1985; and Rossolini et al., Mol. Cell. Probes8:91-98, 1994).

The terms "polypeptide" and "protein" are used interchangeably herein and refer to a polymer of amino acid residues. The terms apply to amino acid polymers in which one or more amino acid residues are artificial chemical mimics of corresponding naturally occurring amino acids, as well as to naturally occurring amino acid polymers and non-naturally occurring amino acid polymers.

The term "naked antibody" refers to an antibody that is not conjugated to a heterologous module (e.g., a cytotoxic module) or a radioactive label. A naked antibody may be present in a pharmaceutical formulation.

The term "identity", "sequence identity" or "amino acid sequence identity" refers to the degree (percentage) to which the amino acids/nucleic acids of two sequences are identical at equivalent positions, when the two sequences are optimally aligned, with gaps introduced as necessary to achieve the maximum percent sequence identity, and without considering any conservative substitutions as part of the sequence identity. To determine percent sequence identity, alignment can be achieved using methods that are well known in the art, such as BLAST, BLAST-2, ALIGN, ALIGN-2 or Megalign (DNASTAR). Those skilled in the art can determine parameters suitable for measuring alignment, including any algorithms required to achieve maximum alignment of the full length of the aligned sequences.

The term "conservatively modified variant" or "conservative substitution" refers to substitutions of amino acids in a protein with other amino acids having similar characteristics (e.g. charge, side-chain size, hydrophobicity/hydrophilicity, backbone conformation and rigidity), such that the changes can be often made without altering the biological activity of the protein. Those skilled in the art recognize that, in general, single amino acid substitutions in non-essential regions of a polypeptide do not substantially alter biological activity (see, e.g., Watson et al. (1987) Molecular Biology of the Gene, The Benjamin/Cummings Pub. Co., p. 224 (4th Ed.)). The term "conservatively modified variant", when applied to nucleic acid sequences, refers to those nucleic acids which encode identical or essentially identical amino acid sequences, or where the nucleic acid does not encode an amino acid sequence, to essentially identical sequences. Because of the degeneracy of the genetic code, a number of functionally identical nucleic acids encode any given protein. For example, the codons GCA, GCC, GCG and GCU all encode the amino acid alanine. Thus, at every position where an alanine is specified by a codon, the codon can be altered to any of the corresponding codons described without altering the encoded polypeptide. Such nucleic acid variations are "silent variations", which are one species of conservatively modified variations. Every nucleic acid sequence herein which encodes a polypeptide also describes every possible silent variation of the nucleic acid. One of skill will recognize that each codon in a nucleic acid (except AUG, which is normally the only codon for methionine, and TGG, which is normally the only codon for tryptophan) can be modified to produce a functionally identical molecule. Thus, each silent variation of a nucleic acid that encodes a polypeptide is implicit in each sequence described.

The term "expression vector" or "expression construct" refers to a vector that is applied to transform a host cell and comprises a nucleic acid sequence that directs and/or controls (along with the host cell) the expression of one or more heterologous coding regions operably linked thereto. Expression constructs may include, but are not limited to, sequences that affect or control transcription and translation and affect RNA splicing of a coding region operably linked thereto in the presence of an intron.

As used herein, "operably linked" means that the components to which the term is applied are in a relationship that allows them to carry out their inherent functions under suitable conditions. For example, a control sequence in a vector that is "operably linked" to a protein-coding sequence is ligated thereto so that expression of the protein-coding sequence is achieved under conditions compatible with the transcriptional activity of the control sequences.

The terms "host cell", "host cell line", and "host cell culture" are used interchangeably and refer to cells into which exogenous nucleic acids have been introduced, including progenies of such cells. Host cells include "transformants" and "transformed cells", which include primary transformed cells and progenies derived therefrom, regardless of the number of passages. Progeny may not be exactly the same as parent cells in terms of nucleic acid content and may contain mutations. Mutant progenies that have the same function or biological activity as the cells screened or selected

from the group consisting of the initially transformed cells are included herein. Host cells include prokaryotic and eukaryotic host cells, wherein eukaryotic host cells include, but are not limited to, mammalian cells, insect cell lines, plant cells, and fungal cells. Exemplary host cells are as follows: Chinese hamster ovary (CHO) cells, NSO, SP2 cells, HeLa cells, baby hamster kidney (BHK) cells, monkey kidney cells (COS), human hepatocellular carcinoma cells (e.g., Hep G2), A549 cells, 3T3 cells and HEK-293 cells, *Pichiapastoris, Pichia finlandica, Candida albicans, Aspergillus niger, Aspergillus oryzae* and *Trichoderma reesei.*

As used in the present application, the expressions "cell", "cell line" and "cell culture" are used interchangeably and include the progeny of such a cell. Thus, the words "transformant" and "transformed cell" include the primary subject cell and cultures derived therefrom, regardless of the passage number. It is also understood that not all progeny have precisely identical DNA contents due to deliberate or inadvertent mutations. Variant progeny that have the same function or biological activity as the original transformed cell from which they were selected are included.

Methods for producing and purifying antibodies and antigen-binding fragments are well known in the art, for example, those described in chapters 5-8 and 15 of "Antibodies: A Laboratory Manual", Cold Spring Harbor Press. The antibody or the antigen-binding fragment described herein is genetically engineered to contain one or more additional human FRs in the non-human CDRs. Human FR germline sequences can be obtained at the website http://imgt.cines.fr of ImMunoGeneTics (IMGT) or from the immunoglobulin journal, 2001ISBN012441351, by comparing the IMGT human antibody variable region germline gene database with the MOE software.

The engineered antibody or antigen-binding fragment of the present disclosure can be prepared and purified by conventional methods. For example, cDNA sequences encoding the heavy and light chains can be cloned and recombined into an expression vector. Recombinant immunoglobulin expression vectors can be stably transfected into host cells. As a more recommended prior art, mammalian expression systems will result in glycosylation of the antibody, particularly at the N-terminal site of the Fc region. Stable clones are obtained by expression of the antibody that specifically binds to human Nectin-4. Positive clones are expanded in a medium in a bioreactor to produce antibodies. The culture medium with the secreted antibody can be purified by conventional techniques, for example, using an A or G Sepharose FF column. Non-specifically bound fractions are washed away. The bound antibody is eluted by the pH gradient method, and the antibody fragments are detected by SDS-PAGE and collected. The antibody can be filtered and concentrated by conventional methods. Soluble mixtures and polymers can also be removed by conventional methods, such as molecular sieves and ion exchange. The resulting product needs to be immediately frozen, e.g., at -70 °C, or lyophilized.

"Isolated" refers to a purified state, and in this case means that the designated molecule is substantially free of other biomolecules, such as nucleic acids, proteins, lipids, carbohydrates, or other materials (such as cell debris and growth medium). Generally, the term "isolated" does not mean the complete absence of such materials or the absence of water, buffers or salts, unless they are present in amounts that will significantly interfere with the experimental or therapeutic use of the compounds described herein.

The term "drug" refers to a chemical substance that can alter or ascertain an organism’s physiology and pathological state and can be used for the prevention, diagnosis and treatment of diseases. The drug includes a cytotoxic drug. There is no clear boundary between a drug and a toxic substance. The toxic substance refers to a chemical substance that has a toxic effect on organisms and can cause damage to human health even in small doses. Any drug in large doses may induce toxic responses.

The cytotoxic drug refers to a substance that inhibits or prevents cell functions and/or causes cell death or cell destruction. The cytotoxic drug can kill tumor cells in principle at a sufficiently high concentration; however, due to lack of specificity, the cytotoxic drug can cause apoptosis of normal cells while killing tumor cells, resulting in serious side effects. The cytotoxic drug includes toxins, such as small molecule toxins or enzymatically active toxins of bacterial, fungal, plant or animal origin, radioisotopes (e.g., $At^{211}$, $I^{131}$, $I^{125}$, $Y^{90}$, $Re^{186}$, $Re^{188}$, $Sm^{153}$, $Bi^{212}$, $P^{32}$ and radioactive isotopes of Lu), chemotherapeutic drugs, antibiotics and nucleolytic enzymes.

The term "alkyl" refers to a saturated aliphatic hydrocarbon group which is a linear or branched group containing 1 to 20 carbon atoms, preferably alkyl containing 1 to 12 (e.g., 1, 2, 3, 4, 5, 6, 7, 8, 9, 10, 11 and 12) carbon atoms, and more preferably alkyl containing 1 to 6 carbon atoms. Non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, n-nonyl, 2-methyl-2-ethylhexyl, 2-methyl-3-ethylhexyl, 2,2-diethylpentyl, n-decyl, 3,3-diethylhexyl, 2,2-diethylhexyl, and various side-chain isomers thereof, and the like. More preferred is a lower alkyl having 1 to 6 carbon atoms, and non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl and the like. Alkyl may be substituted or unsubstituted. When it is substituted, the substituent may be substituted at any available point of attachment, and the substituent is preferably one or more substituents independently optionally selected from the group consisting of D atom, halogen, alkyl, alkoxy, haloalkyl, haloalkoxy, cycloalkyloxy, heterocyclyloxy, hydroxy, hydroxyalkyl, cyano, amino, nitro, cycloalkyl, heterocyclyl, aryl and heteroaryl.

The term "alkoxy" refers to -O-(alkyl), wherein the alkyl is as defined above. Non-limiting examples of alkoxy include: methoxy, ethoxy, propoxy and butoxy. Alkoxy may be optionally substituted or unsubstituted. When it is substituted, the substituent is preferably one or more groups independently selected from the group consisting of D atom, halogen, alkoxy, haloalkyl, haloalkoxy, cycloalkyloxy, heterocyclyloxy, hydroxy, hydroxyalkyl, cyano, amino, nitro, cycloalkyl, heterocyclyl, aryl and heteroaryl.

The term "alkylene" refers to a saturated linear or branched aliphatic hydrocarbon group, which is a residue derived from the parent alkane by removal of two hydrogen atoms from the same carbon atom or two different carbon atoms. It is a linear or branched group containing 1 to 20 carbon atoms, preferably alkylene containing 1 to 12 (e.g., 1, 2, 3, 4, 5, 6, 7, 8, 9, 10, 11 and 12) carbon atoms, and more preferably alkylene containing 1 to 6 carbon atoms. Non-limiting examples of alkylene include, but are not limited to, methylene ($-CH_2-$), 1,1-ethylene ($-CH(CH_3)-$), 1,2-ethylene ($-CH_2CH_2-$), 1,1-propylene ($-CH(CH_2CH_3)-$), 1,2-propylene ($-CH_2CH(CH_3)-$), 1,3-propylene ($-CH_2CH_2CH_2-$), 1,4-butylene ($-CH_2CH_2CH_2CH_2-$), and the like. Alkylene may be substituted or unsubstituted. When it is substituted, the substituent may be substituted at any available point of attachment, and the substituent is preferably one or more substituents independently optionally selected from the group consisting of alkenyl, alkynyl, alkoxy, haloalkoxy, cycloalkyloxy, heterocyclyloxy, alkylthio, alkylamino, halogen, sulfhydryl, hydroxy, nitro, cyano, cycloalkyl, heterocyclyl, aryl, heteroaryl, cycloalkoxy, heterocycloalkoxy, cycloalkylthio, heterocycloalkylthio and oxo.

The term "alkenyl" refers to an alkyl compound having at least one carbon-carbon double bond in the molecule, wherein the alkyl is as defined above. Alkenyl preferably contains 2 to 12 (e.g., 2, 3, 4, 5, 6, 7, 8, 9, 10, 11 and 12) carbon atoms and more preferably contains 2 to 6 carbon atoms. Alkenyl may be substituted or unsubstituted. When it is substituted, the substituent is preferably one or more groups independently selected from the group consisting of alkoxy, halogen, haloalkyl, haloalkoxy, cycloalkyloxy, heterocyclyloxy, hydroxy, hydroxyalkyl, cyano, amino, nitro, cycloalkyl, heterocyclyl, aryl and heteroaryl.

The term "alkynyl" refers to an alkyl compound having at least one carbon-carbon triple bond in the molecule, wherein the alkyl is as defined above. Alkynyl preferably contains 2 to 12 (e.g., 2, 3, 4, 5, 6, 7, 8, 9, 10, 11 and 12) carbon atoms and more preferably contains 2 to 6 carbon atoms. Alkynyl may be substituted or unsubstituted. When it is substituted, the substituent is preferably one or more groups independently selected from the group consisting of alkyl, alkoxy, halogen, haloalkyl, haloalkoxy, cycloalkyloxy, heterocyclyloxy, hydroxy, hydroxyalkyl, cyano, amino, nitro, cycloalkyl, heterocyclyl, aryl and heteroaryl.

The term "cycloalkyl" refers to a saturated or partially unsaturated monocyclic or polycyclic hydrocarbon substituent. The cycloalkyl ring contains 3 to 20 carbon atoms, preferably 3 to 12 (e.g., 3, 4, 5, 6, 7, 8, 9, 10, 11 and 12) carbon atoms, more preferably 3 to 8 carbon atoms, and most preferably 3 to 6 carbon atoms. Non-limiting examples of monocyclic cycloalkyl include cyclopropyl, cyclobutyl, cyclopentyl, cyclopentenyl, cyclohexyl, cyclohexenyl, cyclohexadienyl, cycloheptyl, cycloheptatrienyl, cyclooctyl, and the like. Polycyclic cycloalkyl includes spirocycloalkyl, fused cycloalkyl, and bridged cycloalkyl.

The term "spirocycloalkyl" refers to a 5- to 20-membered polycyclic group in which monocyclic rings share one carbon atom (referred to as the spiro atom), wherein the spirocycloalkyl may contain one or more double bonds. It is preferably 6-14 (e.g., 6, 7, 8, 9, 10, 11, 12, 13 and 14) membered and more preferably 7-10 (e.g., 7, 8, 9 or 10) membered. According to the number of spiro atoms shared among the rings, spirocycloalkyl may be monospirocycloalkyl, bispirocycloalkyl or polyspirocycloalkyl, preferably monospirocycloalkyl and bispirocycloalkyl, and more preferably 3-membered/4-membered, 3-membered/5-membered, 3-membered/6-membered, 4-membered/4-membered, 4-membered/5-membered, 4-membered/6-membered, 5-membered/4-membered, 5-membered/5-membered, 5-membered/6-membered, 6-membered/3-membered, 6-membered/4-membered, 6-membered/5-membered and 6-membered/6-membered monospirocycloalkyl. Non-limiting examples of spirocycloalkyl include:

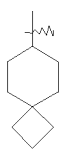 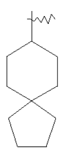 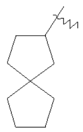 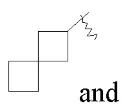 and 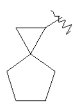 .

The term "fused cycloalkyl" refers to a 5- to 20-membered all-carbon polycyclic group in which each ring shares a pair of adjacent carbon atoms with the other rings in the system, wherein one or more of the rings may contain one

or more double bonds. It is preferably 6-14 (e.g., 6, 7, 8, 9, 10, 11, 12, 13 and 14) membered and more preferably 7-10 (e.g., 7, 8, 9 or 10) membered. According to the number of constituent rings, the fused cycloalkyl may be bicyclic, tricyclic, tetracyclic or polycyclic cycloalkyl, preferably bicyclic or tricyclic cycloalkyl, and more preferably 3-membered/4-membered, 3-membered/5-membered, 3-membered/6-membered, 4-membered/4-membered, 4-membered/5-membered, 4-membered/6-membered, 5-membered/4-membered, 5-membered/5-membered, 5-membered/6-membered, 6-membered/3-membered, 6-membered/4-membered, 6-membered/5-membered and 6-membered/6-membered bicyclic cycloalkyl. Non-limiting examples of fused cycloalkyl include:

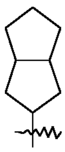 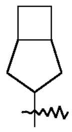 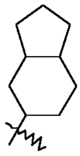 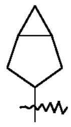 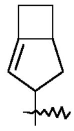 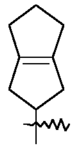 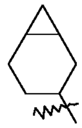 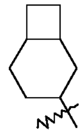 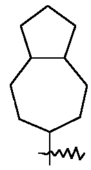

The term "bridged cycloalkyl" refers to a 5- to 20-membered all-carbon polycyclic group in which any two rings share two carbon atoms that are not directly connected, and it may contain one or more double bonds. It is preferably 6-14 (e.g., 6, 7, 8, 9, 10, 11, 12, 13 and 14) membered and more preferably 7-10 (e.g., 7, 8, 9 or 10) membered. According to the number of constituent rings, the bridged cycloalkyl may be bicyclic, tricyclic, tetracyclic or polycyclic, preferably bicyclic, tricyclic or tetracyclic, and more preferably bicyclic or tricyclic. Non-limiting examples of bridged cycloalkyl include:

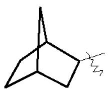 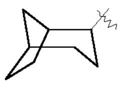 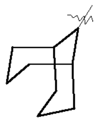 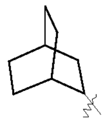 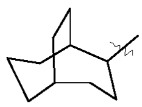

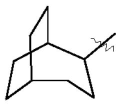 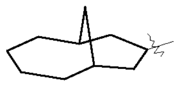 and 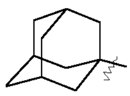 .

The cycloalkyl ring includes those in which the cycloalkyl described above (including monocyclic, spiro, fused and bridged ones) is fused to an aryl, heteroaryl or heterocycloalkyl ring, wherein the ring attached to the parent structure is cycloalkyl. Non-limiting examples include indanyl, tetrahydronaphthyl, benzocycloheptanyl, and the like, and preferably indanyl and tetrahydronaphthyl.

Cycloalkyl may be substituted or unsubstituted. When it is substituted, the substituent may be substituted at any available point of attachment, and the substituent is preferably one or more substituents independently optionally selected from the group consisting of halogen, alkyl, alkoxy, haloalkyl, haloalkoxy, cycloalkyloxy, heterocyclyloxy, hydroxy, hydroxyalkyl, cyano, amino, nitro, cycloalkyl, heterocyclyl, aryl and heteroaryl.

The term "heterocyclyl" refers to a saturated or partially unsaturated monocyclic or polycyclic substituent containing 3 to 20 ring atoms, wherein one or more of the ring atoms are heteroatoms selected from the group consisting of nitrogen, oxygen, sulfur, S(O) and $S(O)_2$, excluding a cyclic portion of -O-O-, -O-S- or -S-S-, and the other ring atoms are carbon atoms. It preferably contains 3 to 12 (e.g., 3, 4, 5, 6, 7, 8, 9, 10, 11 and 12) ring atoms, of which 1 to 4 (e.g., 1, 2, 3 and 4) are heteroatoms; more preferably 3 to 8 (e.g., 3, 4, 5, 6, 7 and 8) ring atoms, of which 1 to 3 (e.g., 1, 2 and 3) are heteroatoms; more preferably 3 to 6 ring atoms, of which 1 to 3 are heteroatoms; most preferably 5 or 6 ring atoms, of which 1 to 3 are heteroatoms. Non-limiting examples of monocyclic heterocyclyl include pyrrolidinyl, tetrahydropyranyl, 1,2,3,6-tetrahydropyridinyl, piperidinyl, piperazinyl, morpholinyl, thiomorpholinyl, homopiperazinyl, and the like. Polycyclic heterocyclyl includes spiroheterocyclyl, fused heterocyclyl, and bridged heterocyclyl.

The term "spiroheterocyclyl" refers to a 5- to 20-membered polycyclic heterocyclyl group in which monocyclic rings share one atom (referred to as the spiro atom), wherein one or more ring atoms are heteroatoms selected from the group consisting of nitrogen, oxygen, sulfur, S(O) and $S(O)_2$, and the other ring atoms are carbon atoms. It may have one or more double bonds. It is preferably 6- to 14-membered, and more preferably 7-to 10-membered (e.g., 7-membered, 8-membered, 9-membered or 10-membered). According to the number of spiro atoms shared among the rings, spiroheterocyclyl may be monospiroheterocyclyl, bispiroheterocyclyl or polyspiroheterocyclyl, preferably monospiroheterocyclyl and bispiroheterocyclyl, and more preferably 4-membered/4-membered, 4-membered/5-membered, 4-membered/6-membered, 5-membered/5-membered, 5-membered/6-membered or 6-membered/6-membered monospiroheterocyclyl. Non-limiting examples of spiroheterocyclyl include:

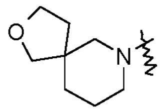 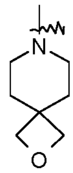 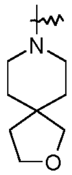 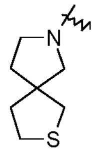 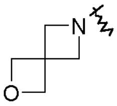 and 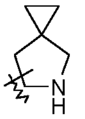 .

The term "fused heterocyclyl" refers to a 5- to 20-membered polycyclic heterocyclyl group in which each ring shares a pair of adjacent atoms with the other rings in the system, wherein one or more of the rings may contain one or more double bonds. In the fused heterocyclyl, one or more of the ring atoms are heteroatoms selected from the group consisting of nitrogen, oxygen, sulfur, S(O) and $S(O)_2$, and the other ring atoms are carbon atoms. It is preferably 6- to 14-membered, and more preferably 7- to 10-membered (e.g., 7-membered, 8-membered, 9-membered or 10-membered). According to the number of constituent rings, the fused heterocyclyl may be bicyclic, tricyclic, tetracyclic or polycyclic fused heterocyclyl, preferably bicyclic or tricyclic fused heterocyclyl, and more preferably 3-membered/4-membered, 3-membered/5-membered, 3-membered/6-membered, 4-membered/4-membered, 4-membered/5-membered, 4-membered/6-membered, 5-membered/4-membered, 5-membered/5-membered, 5-membered/6-membered, 6-membered/3 -membered, 6-membered/4-membered, 6-membered/5-membered and 6-membered/6-membered bicyclic fused heterocyclyl. Non-limiting examples of fused heterocyclyl include:

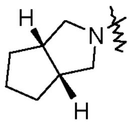 , 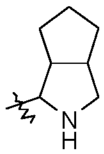 , 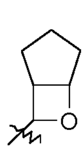 , 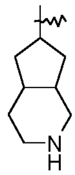 , 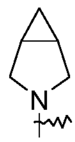 , 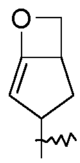 , 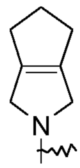 , 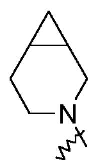 ,

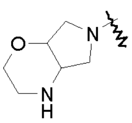 , 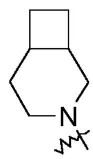 , 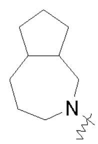 , 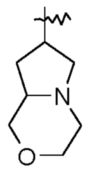 , 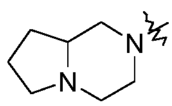 and 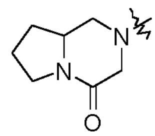 .

The term "bridged heterocyclyl" refers to a 5- to 14-membered polycyclic heterocyclyl group in which any two rings share two carbon atoms that are not directly connected to each other, wherein the bridged heterocyclyl may contain one or more double bonds. In the bridged heterocyclyl, one or more of the ring atoms are heteroatoms selected from the group consisting of nitrogen, oxygen, sulfur, S(O) and $S(O)_2$, and the other ring atoms are carbon atoms. It is preferably 6- to 14-membered, and more preferably 7- to 10-membered (e.g., 7-membered, 8-membered, 9-membered or 10-membered). According to the number of constituent rings, the bridged heterocyclyl may be bicyclic, tricyclic, tetracyclic or polycyclic, preferably bicyclic, tricyclic or tetracyclic, and more preferably bicyclic or tricyclic. Non-limiting examples of bridged heterocyclyl include:

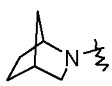 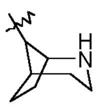 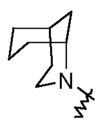 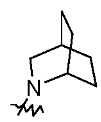

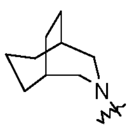 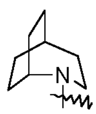 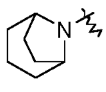 and 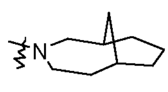 .

The heterocyclyl ring includes those in which the heterocyclyl described above (including monocyclic, spiroheterocyclic, fused heterocyclic and bridged heterocyclic rings) is fused to an aryl, heteroaryl or cycloalkyl ring, wherein

the ring attached to the parent structure is heterocyclyl; its non-limiting examples include:

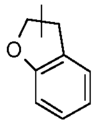, 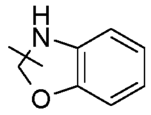, 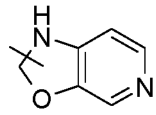, 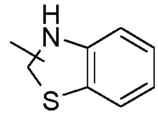,

and the like.

Heterocyclyl may be substituted or unsubstituted. When it is substituted, the substituent may be substituted at any available point of attachment, and the substituent is preferably one or more substituents independently optionally selected from the group consisting of halogen, alkyl, alkoxy, haloalkyl, haloalkoxy, cycloalkyloxy, heterocyclyloxy, hydroxy, hydroxyalkyl, cyano, amino, nitro, cycloalkyl, heterocyclyl, aryl and heteroaryl.

The term "aryl" refers to a 6- to 14-membered, preferably 6- to 10-membered all-carbon monocyclic or fused polycyclic (in which the rings share a pair of adjacent carbon atoms) group having a conjugated $\pi$-electron system, such as phenyl and naphthyl. The aryl ring includes those in which the aryl ring described above is fused to a heteroaryl, heterocyclyl or cycloalkyl ring, wherein the ring attached to the parent structure is the aryl ring; its non-limiting examples include:

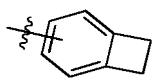, 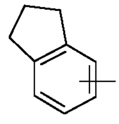, 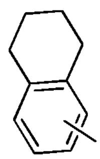, 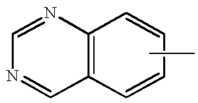, 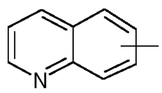, 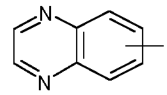,

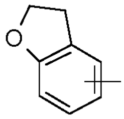, 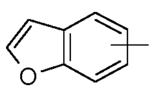, 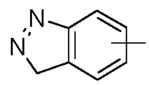, 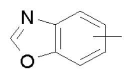, 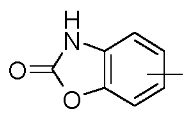, 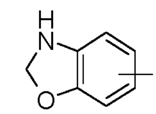,

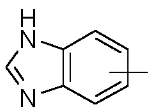, 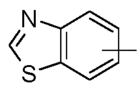, 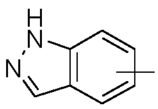, 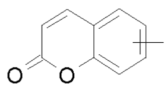 and 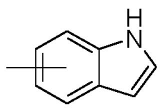.

Aryl may be substituted or unsubstituted. When it is substituted, the substituent may be substituted at any available point of attachment, and the substituent is preferably one or more substituents independently optionally selected from the group consisting of halogen, alkyl, alkoxy, haloalkyl, haloalkoxy, cycloalkyloxy, heterocyclyloxy, hydroxy, hydroxyalkyl, cyano, amino, nitro, cycloalkyl, heterocyclyl, aryl and heteroaryl.

The term "heteroaryl" refers to a heteroaromatic system containing 1 to 4 (e.g., 1, 2, 3, and 4) heteroatoms and 5 to 14 ring atoms, wherein the heteroatoms are selected from the group consisting of oxygen, sulfur and nitrogen. Heteroaryl is preferably 5- to 10-membered (e.g., 5, 6, 7, 8, 9 or 10 membered) and more preferably 5- or 6-membered, e.g., furanyl, thienyl, pyridinyl, pyrrolyl, N-alkylpyrrolyl, pyrimidinyl, pyrazinyl, pyridazinyl, imidazolyl, pyrazolyl, triazolyl and tetrazolyl. The heteroaryl ring includes those in which the heteroaryl ring described above is fused to an aryl, heterocyclyl or cycloalkyl ring, wherein the ring attached to the parent structure is the heteroaryl ring; its non-limiting examples include:

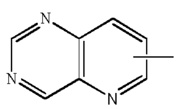, 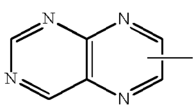, 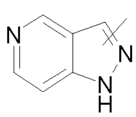, 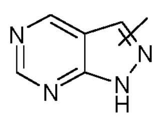, 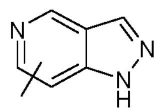, 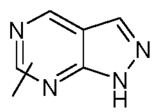,

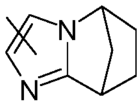, 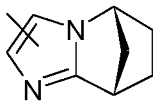, 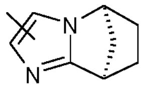, 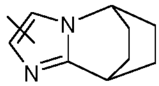, 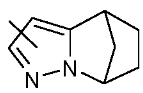, 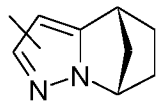,

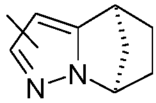, 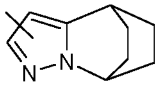, 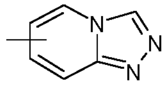, 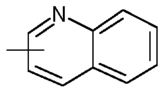, 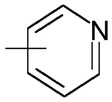, 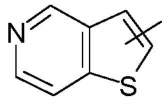,

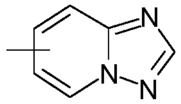, 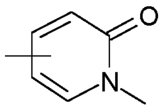, 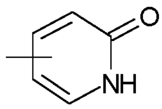, 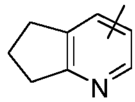, 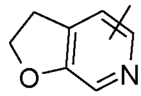,

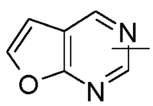, 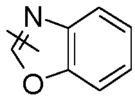, 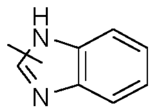, 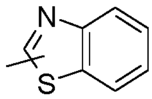, 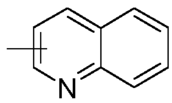, 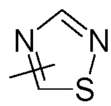,

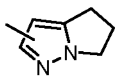, 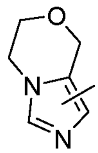, 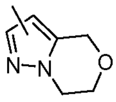 and 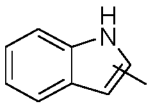.

Heteroaryl may be substituted or unsubstituted. When it is substituted, the substituent may be substituted at any available point of attachment, and the substituent is preferably one or more substituents independently optionally selected from the group consisting of halogen, alkyl, alkoxy, haloalkyl, haloalkoxy, cycloalkyloxy, heterocyclyloxy, hydroxy, hydroxyalkyl, cyano, amino, nitro, cycloalkyl, heterocyclyl, aryl and heteroaryl.

The cycloalkyl, heterocyclyl, aryl and heteroaryl described above include residues derived from the parent ring by removal of one hydrogen atom from a ring atom, or residues derived from the parent ring by removal of two hydrogen atoms from the same ring atom or two different ring atoms, i.e., "divalent cycloalkyl", "divalent heterocyclyl", "arylene" or "heteroarylene".

The term "amino protecting group" refers to a group that can be easily removed and is intended to protect an amino group from being changed when reactions are taking place elsewhere in the molecule. Non-limiting examples include (trimethylsilyl)ethoxymethyl, tetrahydropyranyl, tert-butoxycarbonyl (Boc), acetyl, benzyl, allyl, p-methoxybenzyl, tert-butyldimethylsilyl (TBS), and the like. These groups may be optionally substituted with 1-3 substituents selected from the group consisting of halogen, alkoxy, and nitro.

The term "hydroxy protecting group" refers to a hydroxy derivative that is commonly used to block or protect hydroxy while reactions are taking place on other functional groups of the compound. By way of example, preferably, the hydroxy protecting group may be ($C_{1-10}$ alkyl or aryl)$_3$silyl, e.g., triethylsilyl, triisopropylsilyl, tert-butyldimethylsilyl (TBS) and tert-butyldiphenylsilyl; $C_{1-10}$ alkyl or substituted alkyl, preferably alkoxy or aryl-substituted alkyl, more preferably $C_{1-6}$ alkoxy-substituted $C_{1-6}$ alkyl or phenyl-substituted $C_{1-6}$ alkyl, and most preferably $C_{1-4}$ alkoxy-substituted $C_{1-4}$ alkyl, e.g., methyl, tert-butyl, allyl, benzyl, methoxymethyl (MOM), ethoxyethyl and 2-tetrahydropyranyl (THP); ($C_{1-10}$ alkyl or aryl)acyl, e.g., formyl, acetyl, benzoyl and p-nitrobenzoyl; ($C_{1-6}$ alkyl or 6-membered to 10-membered aryl)sulfonyl; or ($C_{1-6}$ alkoxy or 6-membered to 10-membered aryloxy)carbonyl.

The term "cycloalkyloxy" refers to cycloalkyl-O-, wherein the cycloalkyl is as defined above.

The term "heterocyclyloxy" refers to heterocyclyl-O-, wherein the heterocyclyl is as defined above.

The term "alkylthio" refers to alkyl-S-, wherein the alkyl is as defined above.

The term "haloalkyl" refers to alkyl substituted with one or more halogens, wherein the alkyl is as defined above.

The term "haloalkoxy" refers to alkoxy substituted with one or more halogens, wherein the alkoxy is as defined above.

The term "deuterated alkyl" refers to alkyl substituted with one or more deuterium atoms, wherein the alkyl is as defined above.

The term "hydroxyalkyl" refers to alkyl substituted with one or more hydroxy groups, wherein the alkyl is as

defined above.

The term "halogen" refers to fluorine, chlorine, bromine or iodine.

The term "hydroxy" refers to -OH.

The term "sulfhydryl" refers to -SH.

The term "amino" refers to $-NH_2$.

The term "cyano" refers to -CN.

The term "nitro" refers to $-NO_2$.

The term "oxo" refers to "=O".

The term "carbonyl" refers to C=O.

The term "carboxyl" refers to -C(O)OH.

The term "carboxylate group" refers to -C(O)O(alkyl), -C(O)O(cycloalkyl), (alkyl)C(O)O- or (cycloalkyl)C(O)O-, wherein the alkyl and cycloalkyl are as defined above.

"Antibody-drug conjugate (ADC)" means that an antibody is linked to a biologically active cytotoxin or a small-molecule drug with cell killing activity by a linker unit.

The antibody or the antibody fragment described herein may be coupled to the effector molecule by any means. For example, the antibody or the antibody fragment may be chemically or recombinantly attached to the cytotoxic drug. Chemical means for preparing fusions or conjugates are known in the art and can be used to prepare immunoconjugates. The method for conjugating the antibody or the antibody fragment and the drug must be capable of linking the antibody to the cytotoxic drug without interfering with the ability of the antibody or the antibody fragment to bind to the target molecule.

In one embodiment, both the antibody and cytotoxic drug are proteins and can be coupled using techniques well known in the art. There are hundreds of cross-linking agents disclosed in the art that can conjugate two proteins. The cross-linking agent is generally selected based on reactive functional groups available or inserted on the antibody or cytotoxic drug. Alternatively, if no reactive groups are present, a photo-activatable cross-linking agent may be used. In some cases, it may be desirable to include a spacer between the antibody and the cytotoxic drug. Cross-linking agents known in the art include homobifunctional agents: glutaraldehyde, dimethyl adipimidate and bis(diazobenzidine), and heterobifunctional agents: m-maleimidobenzoyl-N-hydroxysuccinimide and sulfo-m-maleimidobenzoyl-N-hydroxysuccinimide.

Cross-linking agents that can be used to conjugate an effector molecule to an antibody fragment include, for example, TPCH (S-(2-thiopyridyl)-L-cysteine hydrazide) and TPMPH (S-(2-thiopyridyl)sulfhydryl-propionhydrazide). TPCH and TPMPH react on the carbohydrate moiety of the glycoprotein that had previously been oxidized by mild periodate treatment, thereby forming a hydrazone bond between the hydrazide moiety of the crosslinking agent and the aldehyde generated by periodate. The heterobifunctional cross-linking agents GMBS (N-(y-maleimidobutyryloxy)-succinimide) and SMCC (succinimidyl 4-(N-maleimido-methyl)cyclohexane) are reacted with a primary amine, thereby introducing a maleimido group onto the component. This maleimido group may then react with a sulfhydryl group on another component which may be introduced by a cross-linking agent, thereby forming a stable thioether bond between the components. If steric hindrance between the components interferes with the activity of either component, a cross-linking agent may be used to introduce a long spacer between the components, such as N-succinimidyl 3-(2-pyridyldithio)propionate (SPDP). Thus, there are many suitable cross-linking agents that can be used and selected individually depending on their effect on the yield of the optimal immunoconjugate.

Drug loading, also referred to as drug-to-antibody ratio (DAR), refers to the average number of drugs coupled to each antibody in an ADC. It may range, for example, from about 1 to about 10 drugs conjugated to each antibody, and in certain embodiments, from about 1 to about 8 drugs conjugated to each antibody, preferably selected from the group consisting of 2-8, 2-7, 2-6, 2-5, 2-4, 3-4, 3-5, 5-6, 5-7, 5-8 and 6-8 drugs conjugated to each antibody. Illustratively, the drug loading may be an average number from 1, 2, 3, 4, 5, 6, 7, 8, 9 and 10. The ADC general formulas of the present disclosure include a group of antibody-drug conjugates within a certain range as described above. In the embodiments of the present disclosure, drug loading may be represented by n, which is a decimal or an integer. Drug loading can be determined by conventional methods such as UV/visible spectroscopy, mass spectrometry, ELISA assay and HPLC. The term "linker unit" or "linker fragment" refers to a chemical structure fragment or bond, which is linked to an antibody or antigen-binding fragment thereof at one end and to a drug at the other end, and also may be linked to a drug after being linked to another linker.

The term linker may comprise one or more linker components. Exemplary linker components include 6-maleimidocaproyl ("MC"), maleimidopropionyl ("MP"), valine-citrulline ("val-cit" or "vc"), alanine-phenylalanine ("ala-phe"), p-aminobenzyloxycarbonyl ("PAB"), and those derived from coupling to a linker reagent: N-succinimidyl 4-(2-pyridylthio)pentanoate ("SPP"), N-succinimidyl 4-(N-maleimidomethyl)cyclohexane-1 carboxylate ("SMCC", also referred to herein as "MCC"), and N-succinimidyl(4-iodo-acetyl)aminobenzoate ("SIAB"). The linker may include extenders, spacers and amino acid units, and may be synthesized using methods known in the art, such as those described in US2005-0238649A1. The linker may be a "cleavable linker" favoring the release of drugs in cells. For example, acid-labile linkers (e.g., hydrazones), protease-sensitive (e.g., peptidase-sensitive) linkers, photolabile linkers, dimethyl linkers or disulfide-containing linkers can be used (Chari et al., Cancer Research, 52: 127-131(1992); U.S. Patent No. 5,208,020).

Linker components include, but are not limited to:

MC = 6-maleimidocaproyl, whose structure is shown below:

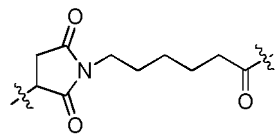

,

Val-Cit or "vc" = valine-citrulline (an exemplary dipeptide in a protease cleavable linker),
citrulline = 2-amino-5-ureidopentanoic acid,
PAB group = p-aminobenzyloxycarbonyl (an example of "self-immolative" linker components), whose structure is shown below:

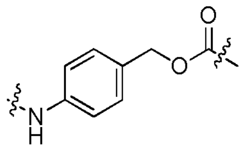

;

Me-Val-Cit = N-methyl-valine-citrulline (where the linker peptide bond has been modified to prevent it from being cleaved by cathepsin B),
MC(PEG)6-OH = maleimidocaproyl-polyethylene glycol (attachable to antibody cysteine),
SPP = N-succinimidyl 4-(2-pyridylthio)valerate,
SPDP = N-succinimidyl 3-(2-pyridyldithio)propionate,
SMCC = succinimidyl-4-(N-maleimidomethyl)cyclohexane-1-carboxylate, and
IT = iminothiolane.

In one embodiment of the present disclosure, the cytotoxic drug is coupled to a sulfhydryl group of the antibody by a linker unit.

The loading of the antibody-drug conjugate can be controlled by the following non-limiting methods, including:

(1) controlling a molar ratio of a linking reagent to a monoclonal antibody,
(2) controlling reaction time and temperature, and
(3) selecting different reaction reagents.

"Optionally" or "optional" means that the event or circumstance subsequently described may, but does not necessarily, occur and that the description includes instances where the event or circumstance occurs or does not occur. For example, "$C_{1-6}$ alkyl that is optionally substituted with a halogen or cyano" means that the halogen or cyano may, but does not necessarily, exist, and the description includes the instance where alkyl is substituted with a halogen or cyano and the instance where alkyl is not substituted with a halogen or cyano.

"Substituted" means that one or more, preferably 1-6, more preferably 1-3 hydrogen atoms in the group are independently substituted with a corresponding number of substituents. Those skilled in the art can determine (experimentally or theoretically) possible or impossible substitution without undue effort. For example, it may be unstable when amino or hydroxy having free hydrogen is bound to a carbon atom having an unsaturated (e.g., olefinic) bond.

"Pharmaceutically acceptable salt" refers to a salt of the compound of the present disclosure, which may be selected from the group consisting of inorganic and organic salts. The salts are safe and effective for use in the body of a mammal and possess the requisite biological activity. The salts may be prepared separately during the final separation and purification of the compound, or by reacting an appropriate group with an appropriate base or acid. Bases commonly used to form pharmaceutically acceptable salts include inorganic bases such as sodium hydroxide and potassium hydroxide, and organic bases such as ammonia. Acids commonly used to form pharmaceutically acceptable salts include inorganic acids and organic acids.

In the present disclosure, the concentration of the antibody-drug conjugate is expressed in terms of the concentration of the protein, i.e., the concentration of the antibody moiety in the antibody-drug conjugate.

For drugs or pharmacologically active agents, the term "therapeutically effective amount" refers to an amount

of a medicament or an agent that is sufficient to provide the desired effect but is non-toxic. The determination of the effective amount varies from person to person. It depends on the age and general condition of a subject, as well as the particular active substance used. The appropriate effective amount in a case may be determined by those skilled in the art in the light of routine tests.

The compounds and intermediates of the present disclosure may also exist in different tautomeric forms, and all such forms are included within the scope of the present disclosure. The term "tautomer" or "tautomeric form" refers to structural isomers of different energies that can interconvert via a low energy barrier. For example, proton tautomers (also known as proton transfer tautomers) include interconversion via proton migration, such as keto-enol and imine-enamine, lactam-lactim isomerization. An example of the lactam-lactim equilibrium is present between A and B as shown below.

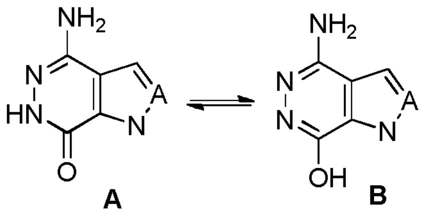

All compounds in the present disclosure can be drawn as form A or form B. All tautomeric forms are within the scope of the present disclosure. The nomenclature of the compounds does not exclude any tautomers.

"Prodrug" refers to a substance that can be converted *in vivo* under physiological conditions, e.g., by hydrolysis in blood, to generate the active prodrug compound.

The term "pharmaceutically acceptable" used herein means that those compounds, materials, compositions and/or dosage forms are, within the scope of reasonable medical judgment, suitable for use in contact with the tissues of subjects without excessive toxicity, irritation, allergic reaction, or other problems or complications, and are commensurate with a reasonable benefit/risk ratio and effective for the intended use. For the preparation of conventional pharmaceutical compositions, refer to Chinese Pharmacopoeia.

As used herein, the singular forms "a", "an" and "the" include plural references and vice versa, unless otherwise clearly defined in the context.

"About" described in the disclosure means that it is within an acceptable error range for a particular value as determined by one of ordinary skill in the art, which will depend in part on how the value is measured or determined, i.e., the limitations of the measurement system. In the context of a particular assay, result or embodiment, "about" means that it is within one standard deviation according to the practice in the art, unless otherwise explicitly stated in the example or elsewhere in the specification.

The term "pharmaceutically acceptable carrier" refers to an ingredient in a pharmaceutical formulation that is different from the active ingredient and is not toxic to the subject. Pharmaceutically acceptable carriers include, but are not limited to, buffers, excipients, stabilizers, or preservatives.

The term "package insert" is used to refer to instructions generally included in commercial packages of therapeutic products, which contain information about the indications, usage, dosage, administration, combination therapy, contraindications and/or warnings concerning the use of such therapeutic products.

The term "subject" or "individual" includes both human and non-human animals. Non-human animals include all vertebrates (e.g., mammals and non-mammals) such as non-human primates (e.g., cynomolgus monkeys), sheep, dogs, cows, chickens, amphibians, and reptiles. Unless indicated, the terms "patient" and "subject" are used interchangeably herein. As used herein, the term "cynomolgus monkey (cyno)" or "cynomolgus" refers to Macaca fascicularis. In certain embodiments, the individual or subject is a human.

The term "excipient" is an addition, besides the main drug, to a pharmaceutical formulation. It may also be referred to as an auxiliary material. For example, binders, fillers, disintegrants and lubricants in tablets; the matrix part in semisolid ointment and cream preparations; preservatives, antioxidants, flavoring agents, fragrances, cosolvents, emulsifiers, solubilizers, osmotic pressure regulators, colorants and the like in liquid formulations can all be referred to as excipients.

The term "diluent", also referred to as a filler, is used primarily to increase the weight and volume of the tablet. The addition of the diluent not only ensures a certain volume, but also reduces the dose deviation of the main ingredients, and improves the drug's compression moldability and the like. When the drug in the tablet form contains oily components, an absorbent is necessarily added to absorb the oily components so as to maintain a "dry" state and thus facilitate the preparation of the tablet. Examples include starch, lactose, inorganic salts of calcium, microcrystalline cellulose and the like.

The term "pharmaceutical composition" refers to a mixture containing one or more of the compounds or the physiologically/pharmaceutically acceptable salts or pro-drugs thereof described herein, and other chemical components, for example, physiologically/pharmaceutically acceptable carriers and excipients. The pharmaceutical composition is intended to promote the administration to an organism, so as to facilitate the absorption of the active ingredient, thereby exerting biological activity.

The pharmaceutical composition may be in the form of a sterile injectable aqueous solution. Available and acceptable vehicles or solvents include water, Ringer's solution and isotonic sodium chloride solution. The sterile injectable formulation may be a sterile injectable oil-in-water microemulsion in which the active ingredient is dissolved in the oil phase. For example, the active ingredient is dissolved in a mixture of soybean oil and lecithin. The oil solution is then added to a mixture of water and glycerol and treated to form a microemulsion. The injection or microemulsion can be locally injected into the bloodstream of a subject in large quantities. Alternatively, it may be desirable to administer the solution and microemulsion in such a way as to maintain a constant circulating concentration of the compound of the present disclosure. To maintain such a constant concentration, a continuous intravenous delivery device may be used. An example of such a device is a Deltec CADD-PLUS. TM. 5400 intravenous injection pump.

The pharmaceutical composition may be in the form of a sterile injectable aqueous or oily suspension for intramuscular and subcutaneous administration. The suspension can be prepared according to the prior art using those suitable dispersants or wetting agents and suspending agents as described above. The sterile injectable formulation may also be a sterile injection or suspension prepared in a parenterally acceptable non-toxic diluent or solvent, e.g., a solution prepared in 1,3-butanediol. In addition, a sterile fixed oil may be conventionally used as a solvent or a suspending medium. For this purpose, any blend fixed oil including synthetic monoglycerides or diglycerides can be used. In addition, fatty acids such as oleic acid may also be used in the preparation of injections. "Administrating" or "giving", when applied to animals, humans, experimental subjects, cells, tissue, organs, or biological fluids, refers to contact of an exogenous drug, a therapeutic agent, a diagnostic agent, or a composition with the animals, humans, subjects, cells, tissue, organs, or biological fluids.

The term "sample" refers to a collection of similar fluids, cells or tissues isolated from a subject, as well as fluids, cells or tissues present within a subject. Exemplary samples are biological fluids (such as blood; serum; serosal fluids; plasma; lymph; urine; saliva; cystic fluids; tears; excretions; sputum; mucosal secretions of secretory tissue and organs; vaginal secretions; ascites; fluids in the pleura; pericardium; peritoneum; abdominal cavity and other body cavities; fluids collected from bronchial lavage; synovial fluids; liquid solutions in contact with a subject or biological source, e.g., cell and organ culture media (including cell or organ conditioned media); lavage fluids; and the like), tissue biopsy samples, fine needle punctures, surgically excised tissues, organ cultures, or cell cultures.

The term "pharmaceutically acceptable salt" refers to a salt of the antibody-drug conjugates of the present disclosure, or a salt of the compound described in the present disclosure. Such salts are safe and effective when used in the body of a mammal and possess the required biological activity. The antibody-drug conjugates of the present disclosure contain at least one amino group and therefore can form salts with acids.

"Treatment" or "treat" (and grammatical variations thereof) refer to clinical intervention in an attempt to alter the natural course of the treated individual, which may be performed either for prophylaxis or during the course of clinical pathology. Desirable effects of the treatment include, but are not limited to, preventing the occurrence or recurrence of a disease, alleviating symptoms, alleviating/reducing any direct or indirect pathological consequences of the disease, preventing metastasis, decreasing the rate of disease progression, ameliorating or alleviating the disease state, and regressing or improving prognosis. In some embodiments, the antibody of the present disclosure is used to delay the development of or slow the progression of a disease.

"Effective amount" is generally an amount sufficient to reduce the severity and/or frequency of symptoms, eliminate symptoms and/or underlying causes, prevent the appearance of symptoms and/or their underlying causes, and/or ameliorate or improve damage (e.g., lung disease) caused by or associated with a disease state. In some embodiments, the effective amount is a therapeutically effective amount or a prophylactically effective amount. "Therapeutically effective amount" is an amount sufficient to treat a disease state or condition, particularly a state or condition associated with the disease state, or to otherwise prevent, hinder, delay, or reverse the progression of the disease state or any other undesirable symptoms associated with the disease in any way. "Prophylactically effective amount" is an amount that, when administered to a subject, will have a predetermined prophylactic effect, e.g., preventing or delaying the onset (or recurrence) of the disease state, or reducing the likelihood of the onset (or recurrence) of the disease state or associated symptoms. Complete treatment or prevention does not necessarily occur after administration of one dose and may occur after administration of a series of doses. Thus, a therapeutically or prophylactically effective amount may be administered in one or more doses. "Therapeutically effective amount" and "prophylactically effective amount" may vary depending on the following factors: e.g., the disease state, age, sex, and weight of the individual, and the ability of a therapeutic agent or combination of therapeutic agents to elicit a desired response in the individual. Exemplary indicators of an effective therapeutic agent or combination of therapeutic agents include, for example, improved health condition of a subject.

One or more embodiments of the present disclosure are described in detail in the specification above. Although any methods and materials similar or identical to those described herein can be used in the practice or testing of the present disclosure, the preferred methods and materials are described below. Other features, objects and advantages of the present disclosure will be apparent from the specification and the claims. In the specification and claims, singular forms include plural referents unless otherwise indicated clearly in the context. Unless otherwise defined, all technical and scientific terms used herein have the meanings generally understood by those of ordinary skill in the art to which the present disclosure belongs. All the patents and publications cited in the specification are incorporated by reference. The following examples are set forth in order to more fully illustrate the preferred embodiments of the present disclosure. These examples should not be construed in any way as limiting the scope of the present disclosure, which is defined by the claims.

**DETAILED DESCRIPTION**

The present invention is further described below with reference to examples, but these examples are not intended to limit the scope of the present invention.

Experimental methods without specific conditions indicated in the examples or test examples of the present disclosure are generally conducted according to conventional conditions, or according to conditions recommended by the manufacturer of the starting materials or commercial products. See Sambrook et al., Molecular Cloning: A Laboratory Manual, Cold Spring Harbor Laboratory Press; Current Protocols in Molecular Biology, Ausubel et al., Greene Publishing Association, Wiley Interscience, NY Reagents without specific origins indicated are commercially available conventional reagents. The application WO2020063676 A1 is incorporated herein by reference in its entirety.

**Examples**

**Example 1: Construction of Cell Strains Highly Expressing Nectin-4**

PBABE-Nectin4 lentiviral expression vector plasmids, pVSV-G and pGag-pol lentiviral system packaging vectors were transfected into viral packaging cells 293T using Lipofectamine 3000 transfection reagent. The medium supernatant containing viruses was collected, filtered, and centrifuged at ultra-high speed. Chinese hamster ovary cells CHO-K1 were allowed to be infected with the concentrated virus, screened using puromycin for two to three weeks, and subjected to FACS single-cell sorting.

According to the Nectin-4 expression levels on the surface of CHO-K1 cells infected with lentivirus determined by FACS, monoclonal cell strains highly expressing Nectin-4 were selected, expanded and cryopreserved for later use.

Amino acid sequence of human Nectin-4 (UniProtKB - Q96NY8-1)

MPLSLGAEMWGPEAWLLLLLLLASFTGRCPAGELETSDVVTVVLGQDAKLPCF
YRGDSGEQVGQVAWARVDAGEGAQELALLHSKYGLHVSPAYEGRVEQPPPPRN
PLDGSVLLRNAVQADEGEYECRVSTFPAGSFQARLRLRVLVPPLPSLNPGPALEE
GQGLTLAASCTAEGSPAPSVTWDTEVKGTTSSRSFKHSRSAAVTSEFHLVPSRSM
NGQPLTCVVSHPGLLQDQRITHILHVSFLAEASVRGLEDQNLWHIGREGAMLKC
LSEGQPPPSYNWTRLDGPLPSGVRVDGDTLGFPPLTTEHSGIYVCHVSNEFSSRD
SQVTVDVLDPQEDSGKQVDLVSASVVVVGVIAALLFCLLVVVVVLMSRYHRRK
AQQMTQKYEEELTLTRENSIRRLHSHHTDPRSQPEESVGLRAEGHPDSLKDNSS

CSVMSEEPEGRSYSTLTTVREIETQTELLSPGSGRAEEEEDQDEGIKQAMNHFVQ
ENGTLRAKPTGNGIYINGRGHLV SEQ ID NO: 1

A positive control antibody EV201 was prepared according to WO2012047724 (page 115). The heavy and light chain amino acid sequences of EV201 (enfortumab vedotin) are as follows:

Heavy chain of EV201:

EVQLVESGGGLVQPGGSLRLSCAASGFTFSSYNMNWVRQAPGKGLEWVSYISS
SSSTIYYADSVKGRFTISRDNAKNSLSLQMNSLRDEDTAVYYCARAYYYGMDV
WGQGTTVTVSSASTKGPSVFPLAPSSKSTSGGTAALGCLVKDYFPEPVTVSWNS
GALTSGVHTFPAVLQSSGLYSLSSVVTVPSSSLGTQTYICNVNHKPSNTKVDKRV
EPKSCDKTHTCPPCPAPELLGGPSVFLFPPKPKDTLMISRTPEVTCVVVDVSHED
PEVKFNWYVDGVEVHNAKTKPREEQYNSTYRVVSVLTVLHQDWLNGKEYKC
KVSNKALPAPIEKTISKAKGQPREPQVYTLPPSREEMTKNQVSLTCLVKGFYPSD
IAVEWESNGQPENNYKTTPPVLDSDGSFFLYSKLTVDKSRWQQGNVFSCSVMH
EALHNHYTQKSLSLSPGK

SEQ ID NO: 2

Light chain of EV201:

DIQMTQSPSSVSASVGDRVTITCRASQGISGWLAWYQQKPGKAPKFLIYAASTL
QSGVPSRFSGSGSGTDFTLTISSLQPEDFATYYCQQANSFPPTFGGGTKVEIK
RTVAAPSVFIFPPSDEQLKSGTASVVCLLNNFYPREAKVQWKVDNALQSGNSQE
SVTEQDSKDSTYSLSSTLTLSKADYEKHKVYACEVTHQGLSSPVTKSFNRGEC

SEQ ID NO: 3

**Example** 2: **Screening for Anti-Human Nectin-4 Monoclonal Antibody**

A positive clone was obtained by panning using a fully human semi-synthetic phage antibody library and antigen Biotinylated Human Nectin-4 (purchased from Beijing ACROBiosystems Biotech Ltd., Cat. # NE4-H82E7) followed by phage detection by ELISA. The positive clone was sequenced. After the sequence was obtained, the positive clone was inserted into the protein expression vector Phr-IgG and expressed on HEK293 and Expi-CHO-S. After purification, FACS and endocytic activity validation assays were performed, and a fully human antibody molecule NEC49 was finally selected.

The variable region sequences of the fully human antibody molecule NEC49 are as follows:
heavy chain variable region of NEC49:

EVQLLESGGGLVQPGGSLRLSCAASGFTFSSYAMSWVRQAPGKGLEWVSAIYS

GGSTYYADSVKGRFTISRDNSKNTLYLQMNSLRAEDTAVYYCTRHGGDSGSWS
YYYYGMDVWGQGTTVTVSS

SEQ ID NO: 6

light chain variable region of NEC49:

DIQMTQSPSSLSASVGDRVTITCRASQSISSYLNWYQQKPGKAPKLLIYAASSLQ
SGVPSRFSGSGSGTDFTLTISSLQPEDFATYYCQQSYSTPLTFGQGTRLEIK

SEQ ID NO: 7

Table 2. CDR sequences obtained by the Chothia numbering scheme

| Antibody | NEC49 | SEQ ID NO |
|---|---|---|
| Heavy chain CDR1 | GFTFSSY | SEQ ID NO: 8 |
| Heavy chain CDR2 | YSGGS | SEQ ID NO: 9 |
| Heavy chain CDR3 | HGGDSGSWSYYYYGMDV | SEQ ID NO: 10 |
| Light chain CDR1 | RASQSISSYLN | SEQ ID NO: 11 |
| Light chain CDR2 | AASSLQS | SEQ ID NO: 12 |
| Light chain CDR3 | QQSYSTPLT | SEQ ID NO: 13 |

Table 3. CDR sequences obtained by the IMGT numbering scheme

| Antibody | NEC49 | SEQ ID NO |
|---|---|---|
| Heavy chain CDR1 | GFTFSSYA | SEQ ID NO: 14 |
| Heavy chain CDR2 | IYSGGST | SEQ ID NO: 15 |
| Heavy chain CDR3 | TRHGGDSGSWSYYYYGMDV | SEQ ID NO: 16 |
| Light chain CDR1 | QSISSY | SEQ ID NO: 17 |
| Light chain CDR2 | AAS | SEQ ID NO: 18 |
| Light chain CDR3 | QQSYSTPLT | SEQ ID NO: 13 |

Table 4. CDR sequences obtained by the Kabat numbering scheme

| Antibody | NEC49 | SEQ ID NO |
|---|---|---|
| Heavy chain CDR1 | SYAMS | SEQ ID NO: 19 |
| Heavy chain CDR2 | AIYSGGSTYYADSVKG | SEQ ID NO: 20 |
| Heavy chain CDR3 | HGGDSGSWSYYYYGMDV | SEQ ID NO: 10 |
| Light chain CDR1 | RASQSISSYLN | SEQ ID NO: 11 |
| Light chain CDR2 | AASSLQS | SEQ ID NO: 12 |
| Light chain CDR3 | QQSYSTPLT | SEQ ID NO: 13 |

Heavy chain constant region of NEC49:

ASTKGPSVFPLAPSSKSTSGGTAALGCLVKDYFPEPVTVSWNSGALTSGVHTFPA
VLQSSGLYSLSSVVTVPSSSLGTQTYICNVNHKPSNTKVDKKVEPKSCDKTHTC
PPCPAPELLGGPSVFLFPPKPKDTLMISRTPEVTCVVVDVSHEDPEVKFNWYVD

GVEVHNAKTKPREEQYNSTYRVVSVLTVLHQDWLNGKEYKCKVSNKALPAPIE
KTISKAKGQPREPQVYTLPPSRDELTKNQVSLTCLVKGFYPSDIAVEWESNGQPE
NNYKTTPPVLDSDGSFFLYSKLTVDKSRWQQGNVFSCSVMHEALHNHYTQKSL
SLSPGK

SEQ ID NO: 4

Light chain constant region of NEC49:

RTVAAPSVFIFPPSDEQLKSGTASVVCLLNNFYPREAKVQWKVDNALQSGNSQE
SVTEQDSKDSTYSLSSTLTLSKADYEKHKVYACEVTHQGLSSPVTKSFNRGEC

SEQ ID NO: 5

Heavy chain of NEC49:

EVQLLESGGGLVQPGGSLRLSCAASGFTFSSYAMSWVRQAPGKGLEWVSAIYS
GGSTYYADSVKGRFTISRDNSKNTLYLQMNSLRAEDTAVYYCTRHGGDSGSWS
YYYYGMDVWGQGTTVTVSSASTKGPSVFPLAPSSKSTSGGTAALGCLVKDYFP
EPVTVSWNSGALTSGVHTFPAVLQSSGLYSLSSVVTVPSSSLGTQTYICNVNHKP
SNTKVDKKVEPKSCDKTHTCPPCPAPELLGGPSVFLFPPKPKDTLMISRTPEVTC
VVVDVSHEDPEVKFNWYVDGVEVHNAKTKPREEQYNSTYRVVSVLTVLHQD
WLNGKEYKCKVSNKALPAPIEKTISKAKGQPREPQVYTLPPSRDELTKNQVSLT
CLVKGFYPSDIAVEWESNGQPENNYKTTPPVLDSDGSFFLYSKLTVDKSRWQQG
NVFSCSVMHEALHNHYTQKSLSLSPGK

SEQ ID NO: 21

Light chain of NEC49:

DIQMTQSPSSLSASVGDRVTITCRASQSISSYLNWYQQKPGKAPKLLIYAASSLQ
SGVPSRFSGSGSGTDFTLTISSLQPEDFATYYCQQSYSTPLTFGQGTRLEIKRTVAA
PSVFIFPPSDEQLKSGTASVVCLLNNFYPREAKVQWKVDNALQSGNSQESVTEQ
DSKDSTYSLSSTLTLSKADYEKHKVYACEVTHQGLSSPVTKSFNRGEC

SEQ ID NO: 22

**Example** 3: **Construction of ADC Molecules ADC drug loading determination**

The drug loading of ADCs was determined by reversed-phase high performance liquid chromatography (RP-HPLC).

**1. Determination method:**

A naked antibody and a test ADC sample (at concentration of 1 mg/mL) were reduced with 4 μL of DDT (sigma) in a water bath at 37 °C for 1 h, and then transferred to an insert. Analysis was performed on a high performance liquid chromatograph Agilent 1200, with Agilent PLRP-S 1000A 8 μm 4.6 × 250 mm selected as the chromatography column, the column temperature at 80 °C, the DAD detector at wavelength 280 nm, the flowrate at 1 mL/min, and the injection volume at 40 μL. Comparisons were made to the spectra of the sample and the naked antibody to identify the locations of the light chain and heavy chain, and then integration was performed on the spectrum of the test sample to calculate the DAR value.

**2. Preparation of solutions**

1) 0.25 M DTT solution:
2) Mobile phase A (0.1% TFA in water):
3) Mobile phase B (0.1% TFA in acetonitrile):

**3. Data analysis**

Comparisons were made to the spectra of the sample and the naked antibody to identify the locations of the light chain and heavy chain, and then integration was performed on the spectrum of the test sample to calculate the drug loading DAR value.

The calculation formula is as follows:

| Name | Number of linked drugs |
|---|---|
| LC | 0 |
| LC+1 | 2 |
| HC | 0 |
| HC+1 | 2 |
| HC+2 | 4 |
| HC+3 | 6 |
| Total LC peak area = LC peak area + LC+1 peak area<br>Total HC peak area = HC peak area + HC+1 peak area + HC+2 peak area + HC+3 peak area<br>LC DAR = Σ(number of linked drugs × percent peak area)/total LC peak area<br>HC DAR = Σ(number of linked drugs × percent peak area)/total HC peak area<br>DAR = LC DAR + HC DAR | |

**Example 4: ADC-1**

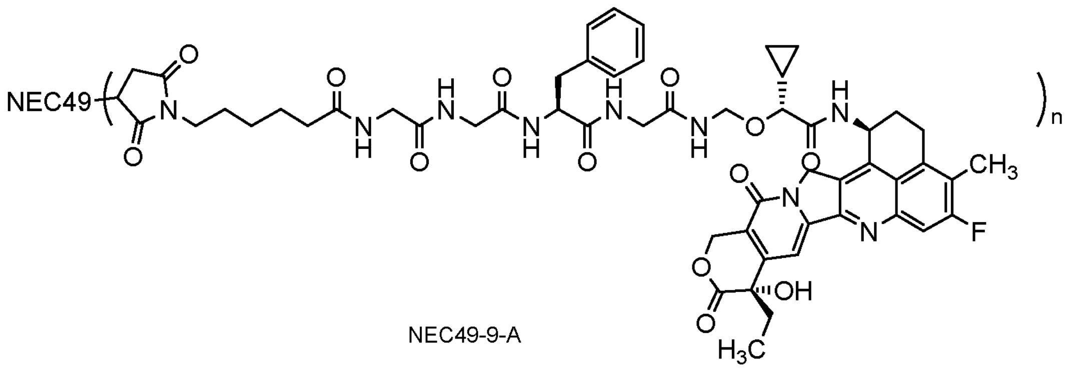

NEC49-9-A

To an aqueous solution of antibody NEC49 in PBS (a pH 6.5, 0.05 M aqueous PBS solution; 10.0 mg/mL, 6.4 mL, 432 nmol), a prepared aqueous solution of tris(2-carboxyethyl)phosphine hydrochloride (TCEP-HCl) (10 mM, 130 μL, 1300 nmol) was added at 37 °C. The mixture was shaken on a water bath shaker at 37 °C for 3 h, and then the reaction was stopped. The reaction mixture was cooled to 25 °C in a water bath.

Compound **9-A** (prepared using the method for compound 9-A of Example 9 on pages 58-60 of WO2020063676, 5.6 mg, 5214 nmol) was dissolved in 300 μL of DMSO, and the resulting solution was added to the above reaction mixture. The mixture was shaken on a water bath shaker at 25 °C for 3 h, and then the reaction was stopped. The reaction mixture was desalted and purified using a Sephadex G25 gel column (elution phase: a pH 6.5, 0.05 M aqueous PBS solution, containing 0.001 M EDTA) to give an exemplary product of the title conjugate general formula NEC49-9-A, **ADC-1,** in PBS (2.89 mg/mL, 21 mL). The product was then stored at 4 °C. Average loading calculated by RP-HPLC: n = 4.90.

**Example 5: ADC-2**

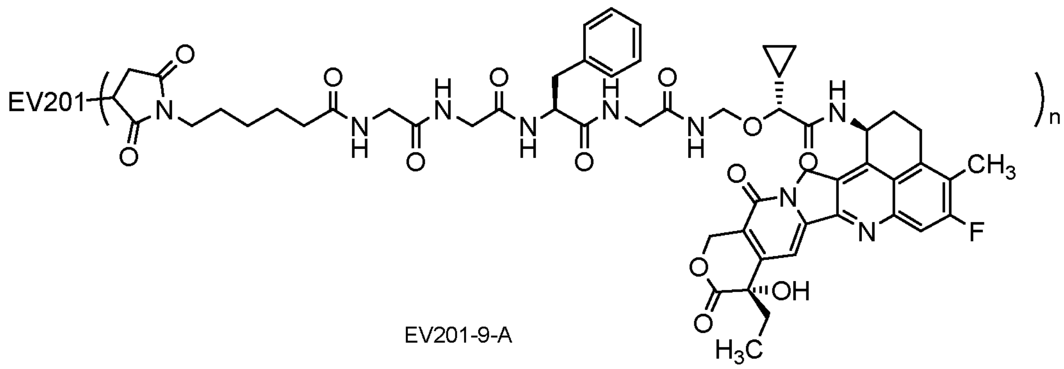

EV201-9-A

To an aqueous solution of antibody EV201 in PBS (a pH 6.5, 0.05 M aqueous PBS solution; 10.0 mg/mL, 1.63 mL, 110 nmol), a prepared aqueous solution of tris(2-carboxyethyl)phosphine hydrochloride (TCEP-HCl) (10 mM, 28.5 μL, 285 nmol) was added at 37 °C. The mixture was shaken on a water bath shaker at 37 °C for 3 h, and then the reaction was stopped. The reaction mixture was cooled to 25 °C in a water bath.

Compound **9-A** (1.18 mg, 1100 nmol) was dissolved in 90 μL of DMSO, and the resulting solution was added to the above reaction mixture. The mixture was shaken on a water bath shaker at 25 °C for 3 h, and then the reaction was stopped. The reaction mixture was desalted and purified using a Sephadex G25 gel column (elution phase: a pH 6.5, 0.05 M aqueous PBS solution, containing 0.001 M EDTA) to give an exemplary product of the title conjugate general formula EV201-9-A, **ADC-2**, in PBS (1.12 mg/mL, 12 mL). The product was then stored at 4 °C. Average loading calculated by RP-HPLC: n = 3.90.

**Example 6: ADC-3**

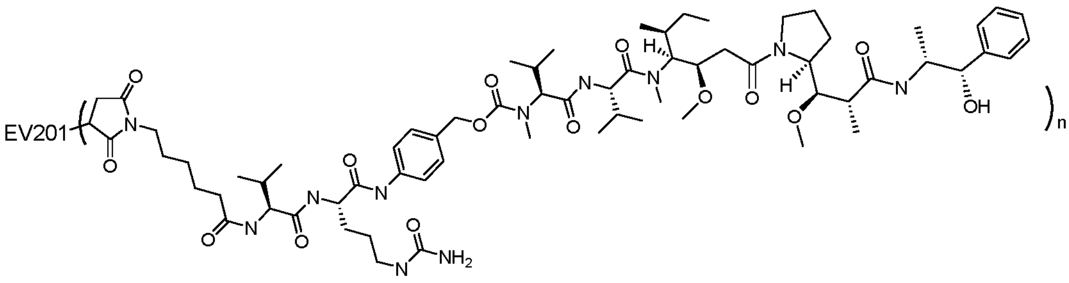

EV201-MMAE

To an aqueous solution of antibody EV201 in PBS (a pH 6.5, 0.05 M aqueous PBS solution; 10.0 mg/mL, 3.67 mL, 248 nmol), a prepared aqueous solution of tris(2-carboxyethyl)phosphine hydrochloride (TCEP-HCl) (10 mM, 64.4 μL, 644 nmol) was added at 37 °C. The mixture was shaken on a water bath shaker at 37 °C for 3 h, and then the reaction was stopped. The reaction mixture was cooled to 25 °C in a water bath.

The compound VcMMAE (Biochempartner, CAS 646502-53-6, 3.3 mg, 2480 nmol) was dissolved in 150 μL of DMSO, and the resulting solution was added to the above reaction mixture. The mixture was shaken on a water bath shaker at 25 °C for 3 h, and then the reaction was stopped. The reaction mixture was desalted and purified using a Sephadex G25 gel column (elution phase: a pH 6.5, 0.05 M aqueous PBS solution, containing 0.001 M EDTA) to give an exemplary product of the title conjugate general formula EV201-MMAE, **ADC-3,** in PBS (2.71 mg/mL, 14 mL). The product was then stored at 4 °C. Average loading calculated by RP-HPLC: n = 4.09.

## Biological Assays

### Test Example 1: Antibody Protein Level Affinity and Kinetics

The anti-Nectin-4 antibody was analyzed by Biacore T200 (GE) for affinity characterization and binding kinetics. A Protein A biosensor chip (Cat. # 29127556, GE) was used to affinity-capture IgG antibodies, and then the human Nectin-4 His (Cat. # 19771-H08H, Sino Biological) antigen that was diluted with HBS-EP buffer (pH 7.4) (Cat. # BR-1001-88, GE) to a series of concentrations was allowed to flow over the surface of the chip. The antigen-antibody binding kinetics was tracked for 3 min and the dissociation kinetics was tracked for 10 min. Reaction signals were detected in real time using a Biacore T200 instrument to obtain binding and dissociation curves. After dissociation was complete in each cycle, the biosensor chip was washed with 10 mM Gly-HCl pH 1.5 (Cat. # BR-1003-54, GE) for regeneration. The data obtained were analyzed with BIAevaluation software of GE using a 1:1 (Langmuir) binding model. The ka (kon), kd (koff) and KD values determined in this way are shown in Table 5.

Table 5. The affinity of antibodies determined by Biacore

| Antibody | ka (1/Ms) | kd (1/s) | KD (M) |
|---|---|---|---|
| EV201 | 7.09E+05 | 6.61E-03 | 9.33E-09 |
| NEC49 | 3.67E+05 | 1.89E-03 | 5.15E-09 |
| Conclusion:<br>NEC49 has a slightly better affinity for human Nectin-4 protein than the control molecule EV201. | | | |

### Test Example 2: *In Vitro* Binding of Antibodies to Cells

Nectin4-CHOK1/T47D/MDA-MB-468 cells were suspended in FACS buffer (2% fetal bovine serum (Gibco, 10099141) pH 7.4 PBS (Sigma, P4417-100TAB)) to form a $1 \times 10^6$/mL cell suspension, and the suspension was then added to a 96-well round-bottom plate at 100 $\mu$L/well. After centrifugation and removal of the supernatant, the test antibodies that were diluted with FACS buffer to different concentrations were added at 50 $\mu$L/well. The plate was incubated away from light in a 4 °C refrigerator for 1 h. The plate was washed 3 times with FACS buffer by centrifugation at 500 g, and Alexa Fluor 488 Goat anti-Human IgG (H+L) (invitrogen, A-11013) was then added in a working concentration. The plate was incubated away from light in a 4 °C refrigerator for 40 min. The plate was washed 3 times with FACS buffer by centrifugation at 500 g and tested on a BD FACSCantoII flow cytometer for geometric mean fluorescence intensity, and the $EC_{50}$ values for the binding of the antibodies to Nectin-4-expressing cells were calculated. The results are shown in Table 6, and FIGs. 1, 2 and 3.

Table 6. The binding activity of antibodies to different cells

| Cell | Nectin4-CHOK1 | | T47D | | MDA-MB-468 | |
|---|---|---|---|---|---|---|
| Antibody | NEC49 | EV201 | NEC49 | EV201 | NEC49 | EV201 |
| Maximal fluorescence value | 82484 | 65807 | 13403 | 12638 | 10191 | 9867 |
| $EC_{50}$ (nM) | 0.78 | 0.94 | 0.07 | 0.26 | 0.06 | 0.23 |
| Conclusion:<br>NEC49 has binding activity for a variety of cell lines expressing Nectin-4 and the binding activity is better than that of the control molecule EV201. | | | | | | |

### Test Example 3: DT3C Antibody Endocytosis Assay

This assay aims to examine the endocytosis of the Nectin-4 antibody according to the killing of cells by activated DT after DT3C protein enters the cells. The *in vitro* endocytic activity of the antibodies was evaluated according to $IC_{50}$ and maximal killing values.

DT3C is a recombinantly expressed fusion protein formed by fusing fragment A of diphtheria toxin (toxin portion only) and fragment 3C of group G streptococcus (IgG binding portion). The protein has a high affinity for the Fc portion of an antibody. It enters cells together with the Fc portion when the antibody is endocytosed, and releases toxic DT under the action of intracellular furin protease. The DT can inhibit the activity of EF2-ADP ribosylation, block the protein

translation process and finally cause cell death. DT3C that does not enter the cell has no activity of cell killing. The endocytic activity of the antibody was evaluated according to cell killing.

A $2 \times 10^4$ cells/mL suspension of Nectin4-CHOK1 cells was prepared with fresh cell medium containing 20% low IgG FBS and added to a cell culture plate 3903 at 50 μL/well. The plate was incubated at 37 °C in 5% carbon dioxide for 16 h. DT3C was diluted to 1.6 μM with serum-free medium, and the antibodies were diluted to 266.4 nM with serum-free medium. 80 μL of DT3C and 80 μL of antibody were mixed (1:1, v/v) and incubated at room temperature for 30 min. The molar concentration of DT3C was 6 times that of the antibodies.

The DT3C-antibody mixture was serially diluted 4-fold with serum-free medium to 8 concentrations. The 9th and 10th points were pure media. C25-IgG1 was an IgG1 isotype negative control group. 50 μL of the diluted mixture was added to 50 μL of cells and incubated in an incubator for three days. To each well, 50 μL of CTG was added. The plate was incubated away from light at room temperature for 10 min. A white membrane was attached to the bottom of the cell culture plate. The plate was placed on a microplate reader Victor 3, and chemiluminescence readings were taken. The results are shown in FIG. 4 (C25-IgG1 as isotype IgG1 negative control, DT3C as negative control) and Table 7.

Table 7. The *in vitro* endocytic activity of antibodies

| Antibody | NEC49 | EV201 |
|---|---|---|
| Maximal killing value | 100.55% | 92.59% |
| $IC_{50}$ (nM) | 0.43 | 0.50 |
| Conclusion: The DT3C assay shows that NEC49 has endocytic activity on Nectin4-CHOK1 cells and the endocytic activity is slightly better than that of the control molecule EV201. | | |

**Test Example 4: pHrodo Antibody Endocytosis Assay**

This assay aims to examine the endocytosis of the Nectin-4 antibody according to changes in fluorescence signal following internalization of the dye. The *in vitro* endocytic activity of the antibody was evaluated according to the intensity of the fluorescent signal.

Fab fragments coupled with the pH sensitive pHrodo iFL dye can bind directly to the Fc region of the Nectin-4 antibody without affecting antibody recognition of the antigen. The pHrodo iFL dye hardly fluoresces at neutral pH. When the Nectin-4 antibody is endocytosed, the dye is internalized at the same time. The fluorescence signal will gradually intensify as the pH decreases. The endocytic activity of the antibody was evaluated according to how the fluorescence signal intensified.

Nectin4-CHOK1 clone 3 cells were cultured with DMEM/F12 + 10% FBS + 10 μg/mL puromycin. On the first day of the experiment, a $2 \times 10^5$ cells/mL cell suspension was prepared with fresh cell-containing medium and added to a 96-well cell culture plate 3903 at 100 μL/well. The plate was cultured at 37 °C in 5% carbon dioxide for 24 h.

4× antibody preparation: The antibody stock solution was diluted 10-fold in FBS-free medium to make a medium solution, and the medium solution was diluted to make a 4× dosing solution (80 nM), with a final antibody concentration of 20 nM.

4× Zenon™ pHrodo™ iFL IgG labeling reagent preparation: The pHrodo™ labeling reagent stock solution was diluted with FBS-free medium to 4× concentration (240 nM), with a final concentration of 60 nM. The above 4× antibody solution and 4× pHrodo™ labeling reagent solution were mixed together in equal volumes and incubated at room temperature for 10 min. 50 μL of the mixture was added to 150 μL of serum-free medium, so that each antibody sample had two concentrations (20 nM and 5 nM).

50 μL of the cell broth was removed from the 3903 plate, and 50 μL of a mixture of antibody and pHrodo dye was added to each well. Two replicate wells were set for each antibody sample. A dye addition-only group and an isotype IgG1 control group were set. After 24 hours of culture in an incubator, the medium was removed, and the cells in each well were digested with 50 μL of pancreatin for 2 min. The digestion was stopped with 50 μL of fresh medium. The cells from the replicate wells of the same sample were transferred to a well of a round-bottom plate using a multi-channel pipette. The cells were centrifuged at 1500 rpm for 2 min, and the medium was discarded. The cells were washed once with FACS buffer (PBS + 2.5%FBS) and centrifuged at 1500 rpm for 2 min. The cells were resuspended in 200 μL of FACS buffer (PBS + 2.5% FBS), and FITC signals were detected using a flow cytometer. Data were analyzed by Flowjo 7.6. The endocytosis results of NEC49 and EV201 on Nectin4-CHOK1 clone3 (C25-IgG1 as IgG1 isotype negative control) are shown in Table 8, FIG. 5A and FIG. 5B.

Table 8. The *in vitro* endocytic activity of antibodies

| Sample | 20 nM antibody + 60 nM pHrodo | 5 nM antibody + 15 nM pHrodo |
|---|---|---|
| | FITC-A signal value | FITC-A signal value |
| NEC49 | 1084 | 303 |
| EV201 | 862 | 281 |
| C25-IgG1 | 154 | 129 |
| pHrodo, antibody-free | 160 | 127 |
| Medium | 11 | 131 |

The pHrodo assay shows that NEC49 has endocytic activity on Nectin4-CHOK1 cells and the endocytic activity is better than that of the control molecule EV201.

**Test Example 5: Cell Activity of ADC Molecules**

This assay aims to examine the killing effects of Nectin4-ADC samples on cells and to evaluate the *in vitro* activity of Nectin4-ADC according to $IC_{50}$ and maximal kill values. T47D (human mammary gland ductal carcinoma cell, ATCC® HTB-133™), MDA-MB-468 (human breast cancer cell, ATCC® HTB-132™) and MDA-MB-231 (human breast cancer cell, ATCC® HTB-26™) cells were digested with pancreatin, neutralized with fresh medium, centrifuged at 1000 rpm, and then resuspended in medium. After counting, the cell suspension was adjusted to a density of 3703 cells/mL and added to a 96-well cell culture plate 3903 at 135 μL/well. Cells were not added to column 11, and only 135 μL of medium was added. The plate was incubated at 37 °C in 5% carbon dioxide for 16 h.

With the concentration of the stock solutions of the samples as the initial concentration, ADC-1 and ADC-2 were diluted five-fold with PBS to 8 concentrations. The cell culture plate was taken out, and 15 μL of the 10× solution was added to each well. The cells were cultured at 37 °C in 5% carbon dioxide for 6 days.

To each well, 70 μL of CTG was added. The plate was incubated away from light at room temperature for 10 min. A white membrane was attached to the bottom of the cell culture plate. The plate was placed on Victor3, and chemiluminescence readings were taken. The data from this assay was processed using the data processing software GraphPad prism5.0. The results are shown in Table 9.

Table 9. The killing of cells by Nectin4-ADC drugs

| **Sample name** | T47D (+++) $IC_{50}$(nM) | MDA-MB-468 (++) $IC_{50}$(nM) | MDA-MB-231 (-) $IC_{50}$(nM) |
|---|---|---|---|
| ADC-1 | 111.20 | 87.52 | 1758.00 |
| ADC-2 | 192.60 | 151.50 | 967.60 |
| Note: + represents the expression level of Nectin4 on cells: the more +, the higher the expression level. NEC49-9-A (ADC-1) showed a significant killing effect in cells with moderate-to-high expression, and the killing was significantly lower in cells with low expression and no expression. This indicates that the molecule is more selective than ADC-2. | | | |

**Test Example 6: Evaluation *of In Vivo* Efficacy of ADC Molecules Against T47D Xenograft Tumors in Mice**

Estrogen tablets (0.36 mg/tablet) were subcutaneously inoculated into the left back of each Balb/c nude mouse. After three days, 0.2 mL ($10 \times 10^6$) of T47D cells (with matrigel, 1: 1 (v/v)) were subcutaneously inoculated into the right back of each mouse. When the mean tumor volume reached about 150 $mm^3$, mice were divided into 5 groups of 8 and administration was started.

Three doses of an ADC compound (prepared in PBS) were intravenously injected into each mouse at 10 μL/g body weight. The blank solvent group was injected with PBS.

The tumor volumes and body weights were measured twice a week and the results were recorded. Data were recorded using Excel statistical software: the average values were calculated as avg; the SD values were calculated as STDEV; the SEM values were calculated as STDEV/SQRT (number of animals per group); GraphPad Prism software was used for plotting, and statistical analysis of the data was performed using Two-way ANOVA or One-way ANOVA.

Tumor volume (V) was calculated as: $V = 1/2 \times L_{long} \times L_{short}^2$

Relative tumor proliferation rate T/C (%) = (T - $T_0$)/(C - $C_0$) × 100%, where T and C are the tumor volumes of animals at the end of the experiment in the treatment group and control group, respectively; $T_0$ and $C_0$ are the tumor volumes of animals at the beginning of the experiment in the treatment group and control group, respectively.

$$\text{Tumor growth inhibition TGI (\%)} = 1 - \text{T/C (\%)}.$$

The results are shown in Table 10 and FIG. 6.

Table 10. The efficacy of ADCs against T47D xenograft tumors in tumor-bearing nude mice

| ADC | Tumor growth inhibition TGI (%) | |
|---|---|---|
| | 5mpk | 2.5mpk |
| ADC-3 | 101 | 73 |
| ADC-1 | 121 | 97 |

In this assay, the test samples ADC-3 and ADC-1 showed significant inhibitory effects on the growth of the xenograft tumors of human breast cancer T47D cells in mice, and the effects were significantly dose-dependent. ADC-1 has a better inhibitory effect on tumor growth than the control molecule in identical doses.

**Test Example 7: Toxicity of ADC Molecule in Rats**

SD rats (male, Zhejiang Vital River Laboratory Animal Technology Co., Ltd.) were injected with ADC compound (prepared in normal saline) through tail vein. The 4 rats in each group were administered ADC compound once a week for 3 weeks, and each was injected 5 mL/kg body weight.

Experimental design:

Table 11. Administration regimen for toxicity tests

| Group | ADC compound | Dose (mg/kg) |
|---|---|---|
| Vehicle | Normal saline | 0 |
| A | ADC-3 | 50 |
| B | ADC-1 | 50 |

The results show that: in group A, animal death, cessation of eating, and rapid body weight loss occurred after the first administration; the experiment was immediately stopped, and adrenomegaly and intussusception were observed upon dissection. In group B, following three consecutive administrations, no abnormalities were found during clinical animal observations, upon dissection and in the blood coagulation function. The results are shown in Table 12.

Table 12. The toxicity test results for ADC compounds in SD rats

| Indicators of abnormality | Hematological indexes (mean ± SD) | | | Blood biochemical indexes (mean ± SD) | | Organ indexes (%, mean ± SD) | |
|---|---|---|---|---|---|---|---|
| 50mpk | WBC (*$10^9$/L) | RET (*$10^9$/L) | LYM (*$10^9$/L) | ALT (IU/L) | TBIL (umol/L) | Thymus | Liver |
| Normal saline | 10.6±0.2 | 306.6±12.1 | 9.5±0.3 | 31.9±3.6 | 0.6±0.4 | 0.2±0.02 | 3.3±0.7 |
| ADC-3 | 2.7±0.7 | 49.7±14.5 | 2.5±0.7 | 422.8±51.0 | 36.1±11.0 | 0.2±0.01 | 4.5±0.04 |

(continued)

| Indicators of abnormality | Hematological indexes (mean ± SD) | | | Blood biochemical indexes (mean ± SD) | | Organ indexes (%, mean ± SD) | |
|---|---|---|---|---|---|---|---|
| ADC-1 | 9.8±1.8 | 273.9±52.6 | 8.2±1.5 | 44.5±7.3 | 0.3±0.1 | 0.07±0.02 | 3.2±0.07 |
| Note: WBC (white blood cells), RET# (reticulocytes), LYM (lymphocytes), ALT (glycine aminotransferase), TBIL (total bilirubin)<br>Conclusion: | | | | | | | |

The ADC-3 group showed animal death after the first administration and abnormalities in several hematological indexes and blood biochemical indexes. After the ADC-1 group finished three consecutive administrations, no abnormalities were found during clinical animal observations, upon dissection and in the blood coagulation function, and

no significant abnormalities were detected in these indicators except for a significant reduction in the size of the thymus.

The preliminary results of the toxicity test in rats suggest that ADC-1 is safer *in vivo* and has lower toxic and side effects than the control molecule.

**Test Example 8: Pharmacokinetics of ADC Molecule**

One injection was intravenously administered to each F344 RG rat (male, Beijing Vitalstar Biotechnology Co., Ltd.) from groups of 4 at 5 mL/kg body weight or 10 mg/kg body weight. 0.2 mL whole blood samples were collected before the administration and at 5 min, 8 h, 1 d, 2 d, 4 d, 7 d, 10 d, 14 d, 21 d and 28 d after the administration, left at 4 °C for 30 min (without anticoagulation), and centrifuged at 1000 g for 15 min. The supernatants (serum) were placed into EP tubes and stored at -80 °C. The antibody (total antibody) concentration and intact ADC concentration in the serum were determined by ELISA. The results are shown in FIG. 7 and Table 13.

Results:

Table 13. The PK half-life and primary pharmacokinetic parameters in F344 RG rats

| ADC-1 10mpk | Total antibody | Intact ADC |
|---|---|---|
| T1/2 (days) | 3.5 ± 0.7 | 3.1 ± 0.6 |
| AUC 0-t (μg/mL*h) | 18998.27 | 18176.38 |
| Cmax (μg/mL) | 346.11 | 380.57 |
| CL (mL/day/kg) | 12.41 | 13.15 |
| AUC intact ADC/total antibody | 95.7% | |

The results show that ADC-1 has relatively good plasma stability in rats.

**Claims**

1. An anti-Nectin-4 antibody, wherein the anti-Nectin-4 antibody binds to Nectin-4 protein expressed by T47D cells or MDA-MB-468 cells with an $EC_{50}$ value of less than 0.1 nM, as determined by FACS.

2. The anti-Nectin-4 antibody according to claim 1, wherein the anti-Nectin-4 antibody comprises a HCDR1, a HCDR2 and a HCDR3 contained in a heavy chain variable region set forth in SEQ ID NO: 6; and a LCDR1, a LCDR2 and a LCDR3 contained in a light chain variable region set forth in SEQ ID NO: 7;
preferably, the anti-Nectin-4 antibody comprises a heavy chain variable region and a light chain variable region, wherein:

    a. the heavy chain variable region comprises a HCDR1, a HCDR2 and a HCDR3 set forth in SEQ ID NO: 8,

SEQ ID NO: 9 and SEQ ID NO: 10, respectively; and the light chain variable region comprises a LCDR1, a LCDR2 and a LCDR3 set forth in SEQ ID NO: 11, SEQ ID NO: 12 and SEQ ID NO: 13, respectively;
the HCDR1-3 and LCDR1-3 described above are determined according to the Chothia numbering scheme; or
b. the heavy chain variable region comprises a HCDR1, a HCDR2 and a HCDR3 set forth in SEQ ID NO: 14, SEQ ID NO: 15 and SEQ ID NO: 16, respectively; and the light chain variable region comprises a LCDR1, a LCDR2 and a LCDR3 set forth in SEQ ID NO: 17, SEQ ID NO: 18 and SEQ ID NO: 13, respectively;
the HCDR1-3 and LCDR1-3 described above are determined according to the IMGT numbering scheme; or
c. the heavy chain variable region comprises a HCDR1, a HCDR2 and a HCDR3 set forth in SEQ ID NO: 19, SEQ ID NO: 20 and SEQ ID NO: 10, respectively; and the light chain variable region comprises a LCDR1, a LCDR2 and a LCDR3 set forth in SEQ ID NO: 11, SEQ ID NO: 12 and SEQ ID NO: 13, respectively;

the HCDR1-3 and LCDR1-3 described above are determined according to the Kabat numbering scheme.

**3.** An anti-Nectin-4 antibody, wherein the anti-Nectin-4 antibody comprises a heavy chain variable region and a light chain variable region, wherein:

a. the heavy chain variable region comprises a HCDR1, a HCDR2 and a HCDR3 set forth in SEQ ID NO: 8, SEQ ID NO: 9 and SEQ ID NO: 10, respectively; and the light chain variable region comprises a LCDR1, a LCDR2 and a LCDR3 set forth in SEQ ID NO: 11, SEQ ID NO: 12 and SEQ ID NO: 13, respectively;
the HCDR1-3 and LCDR1-3 described above are determined according to the Chothia numbering scheme; or
b. the heavy chain variable region comprises a HCDR1, a HCDR2 and a HCDR3 set forth in SEQ ID NO: 14, SEQ ID NO: 15 and SEQ ID NO: 16, respectively; and the light chain variable region comprises a LCDR1, a LCDR2 and a LCDR3 set forth in SEQ ID NO: 17, SEQ ID NO: 18 and SEQ ID NO: 13, respectively;
the HCDR1-3 and LCDR1-3 described above are determined according to the IMGT numbering scheme; or
c. the heavy chain variable region comprises a HCDR1, a HCDR2 and a HCDR3 set forth in SEQ ID NO: 19, SEQ ID NO: 20 and SEQ ID NO: 10, respectively; and the light chain variable region comprises a LCDR1, a LCDR2 and a LCDR3 set forth in SEQ ID NO: 11, SEQ ID NO: 12 and SEQ ID NO: 13, respectively;

the HCDR1-3 and LCDR1-3 described above are determined according to the Kabat numbering scheme.

**4.** The anti-Nectin-4 antibody according to any one of claims 1 to 3, wherein the anti-Nectin-4 antibody is a human antibody or an antigen-binding fragment thereof.

**5.** The anti-Nectin-4 antibody according to any one of claims 1 to 4, comprising a heavy chain variable region and a light chain variable region, wherein:

the heavy chain variable region has at least 90% sequence identity to SEQ ID NO: 6, and/or the light chain variable region has at least 90% sequence identity to SEQ ID NO: 7;
preferably,
the heavy chain variable region is set forth in SEQ ID NO: 6, and the light chain variable region is set forth in SEQ ID NO: 7.

**6.** The anti-Nectin-4 antibody according to any one of claims 1 to 5, wherein the anti-Nectin-4 antibody comprises:

a heavy chain having at least 85% sequence identity to SEQ ID NO: 21, and/or a light chain having at least 85% sequence identity to SEQ ID NO: 22;
preferably, the anti-Nectin-4 antibody comprises:
a heavy chain set forth in SEQ ID NO: 21 and a light chain set forth in SEQ ID NO: 22.

**7.** The anti-Nectin-4 antibody according to any one of claims 3 to 6, wherein the anti-Nectin-4 antibody binds to Nectin-4 protein expressed by T47D cells or MDA-MB-468 cells with an $EC_{50}$ value of less than 0.1 nM, as determined by FACS.

**8.** An anti-Nectin-4 antibody, wherein the antibody competes for binding to human Nectin-4 with the anti-Nectin-4 antibody according to any one of claims 1 to 7.

**9.** A nucleic acid molecule encoding the anti-Nectin-4 antibody according to any one of claims 1 to 8.

**10.** A host cell comprising the nucleic acid molecule according to claim 9.

**11.** An immunoconjugate comprising the anti-Nectin-4 antibody according to any one of claims 1 to 8 and an effector molecule, wherein the effector molecule is coupled to the anti-Nectin-4 antibody; preferably, the effector molecule is selected from the group consisting of an anti-tumor agent, an immunomodulator, a biological response modifier, a lectin, a cytotoxic drug, a chromophore, a fluorophore, a chemiluminescent compound, an enzyme, a metal ion, and any combination thereof.

**12.** A method for immunodetection or determination of Nectin-4 *in vivo* and/or *in vitro,* wherein the method comprises a step of contacting the anti-Nectin-4 antibody according to any one of claims 1 to 8 with a subject or a sample from the subject.

**13.** An antibody-drug conjugate of general formula (Pc-L-Y-D) or a pharmaceutically acceptable salt thereof:

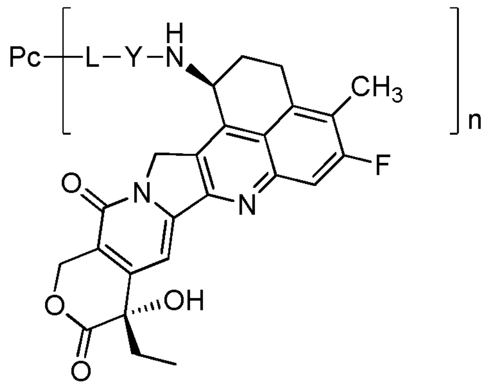

(Pc-L-Y-D)

wherein:

Y is selected from the group consisting of -O-$(CR^aR^b)_m$-$CR^1R^2$-C(O)-, -O-$CR^1R^2$-$(CR^aR^b)_m$-, -O-$CR^1R^2$-, -NH-$(CR^aR^b)_m$-$CR^1R^2$-C(O)- and -S-$(CR^aR^b)_m$-$CR^1R^2$-C(O)-;
wherein $R^a$ and $R^b$ are identical or different and are each independently selected from the group consisting of hydrogen, deuterium, halogen, alkyl, haloalkyl, deuterated alkyl, alkoxy, hydroxy, amino, cyano, nitro, hydroxyalkyl, cycloalkyl and heterocyclyl; or $R^a$ and $R^b$, together with the carbon atom to which they are attached, form cycloalkyl or heterocyclyl;
$R^1$ is selected from the group consisting of halogen, haloalkyl, deuterated alkyl, cycloalkyl, cycloalkylalkyl, alkoxyalkyl, heterocyclyl, aryl and heteroaryl; $R^2$ is selected from the group consisting of hydrogen, halogen, haloalkyl, deuterated alkyl, cycloalkyl, cycloalkylalkyl, alkoxyalkyl, heterocyclyl, aryl and heteroaryl; or $R^1$ and $R^2$, together with the carbon atom to which they are attached, form cycloalkyl or heterocyclyl;
or $R^a$ and $R^2$, together with the carbon atom to which they are attached, form cycloalkyl or heterocyclyl;
m is an integer from 0 to 4;
n is from 1 to 10;
L is a linker unit;
Pc is the anti-Nectin-4 antibody according to any one of claims 1 to 8.

**14.** The antibody-drug conjugate of general formula (Pc-L-Y-D) or the pharmaceutically acceptable salt thereof according to claim 13, wherein n is from 1 to 8; preferably, n is from 2 to 8.

**15.** The antibody-drug conjugate of general formula (Pc-L-Y-D) or the pharmaceutically acceptable salt thereof according to claim 13 or 14,
wherein:

Y is -O-$(CR^aR^b)_m$-$CR^1R^2$-C(O)-;
wherein $R^a$ and $R^b$ are identical or different and are each independently selected from the group consisting of hydrogen, deuterium, halogen and $C_{1-6}$ alkyl;
$R^1$ is $C_{1-6}$ haloalkyl or $C_{3-6}$ cycloalkyl;

$R^2$ is selected from the group consisting of hydrogen, $C_{1-6}$ haloalkyl and $C_{3-6}$ cycloalkyl;
or $R^1$ and $R^2$, together with the carbon atom to which they are attached, form $C_{3-6}$ cycloalkyl;
m is 0 or 1.

**16.** The antibody-drug conjugate of general formula (Pc-L-Y-D) or the pharmaceutically acceptable salt thereof according to any one of claims 13 to 15, wherein Y is selected from the group consisting of:

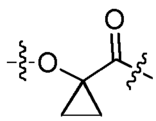, 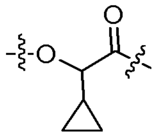, 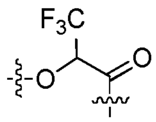, 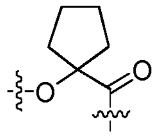, 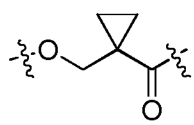, 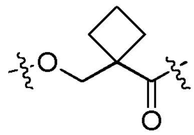

and

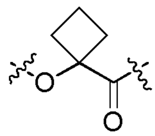;

wherein the O-terminus of Y is connected to the linker unit L.

**17.** The antibody-drug conjugate of general formula (Pc-L-Y-D) or the pharmaceutically acceptable salt thereof according to any one of claims 13 to 16, wherein the linker unit -L- is $-L^1-L^2-L^3-L^4-$, wherein

$L^1$ is selected from the group consisting of -(succinimidyl-3-yl-*N*)-W-C(O)-, $-CH_2-C(O)-NR^3-W-C(O)-$ and -C(O)-W-C(O)-, wherein W is selected from the group consisting of $C_{1-6}$ alkylene and $C_{1-6}$ alkylene-$C_{3-6}$ cycloalkyl, wherein the $C_{1-6}$ alkylene or $C_{1-6}$ alkylene-$C_{3-6}$ cycloalkyl are each independently optionally further substituted with one or more substituents selected from the group consisting of halogen, hydroxy, cyano, amino, $C_{1-6}$ alkyl, $C_{1-6}$ haloalkyl, deuterated $C_{1-6}$ alkyl, $C_{1-6}$ alkoxy and $C_{3-6}$ cycloalkyl;
$L^2$ is selected from the group consisting of $-NR^4(CH_2CH_2O)p^1CH_2CH_2C(O)-$, $-NR^4(CH_2CH_2O)p^1CH_2C(O)-$, $-S(CH_2)p^1C(O)-$ and a chemical bond, wherein $p^1$ is an integer from 1 to 20;
$L^3$ is a peptide residue consisting of 2 to 7 amino acid residues, wherein the amino acid residues are selected from the group consisting of amino acid residues formed from amino acids from phenylalanine, alanine, glycine, valine, lysine, citrulline, serine, glutamic acid and aspartic acid, and are optionally further substituted with one or more substituents selected from the group consisting of halogen, hydroxy, cyano, amino, alkyl, chloroalkyl, deuterated alkyl, alkoxy and cycloalkyl;
$L^4$ is selected from the group consisting of $-NR^5(CR^6R^7)_t-$, $-C(O)NR^5-$, $-C(O)NR^5(CH_2)_t-$ and a chemical bond, wherein t is an integer from 1 to 6;
$R^3$, $R^4$ and $R^5$ are identical or different and are each independently selected from the group consisting of hydrogen, $C_{1-6}$ alkyl, $C_{1-6}$ haloalkyl, deuterated $C_{1-6}$ alkyl and $C_{1-6}$ hydroxyalkyl;
$R^6$ and $R^7$ are identical or different and are each independently selected from the group consisting of hydrogen, halogen, $C_{1-6}$ alkyl, $C_{1-6}$ haloalkyl, deuterated $C_{1-6}$ alkyl and $C_{1-6}$ hydroxyalkyl.

**18.** The antibody-drug conjugate of general formula (Pc-L-Y-D) or the pharmaceutically acceptable salt thereof according to any one of claims 13 to 17, wherein the linker unit -L- is $-L^1-L^2-L^3-L^4-$, wherein:

$L^1$ is

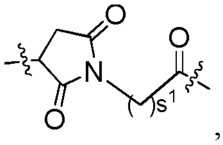,

wherein $s^1$ is an integer from 2 to 8;
$L^2$ is a chemical bond;

$L^3$ is a tetrapeptide residue; preferably, $L^3$ is a tetrapeptide residue set forth in SEQ ID NO: 23;
$L^4$ is $-NR^5(CR^6R^7)t-$, wherein $R^5$, $R^6$ and $R^7$ are identical or different and are each independently hydrogen or $C_{1-6}$ alkyl, and t is 1 or 2;
the $L^1$ terminus of -L- is connected to Pc, and the $L^4$ terminus is connected to Y

**19.** The antibody-drug conjugate of general formula (Pc-L-Y-D) or the pharmaceutically acceptable salt thereof according to any one of claims 13 to 18, wherein -L- is:

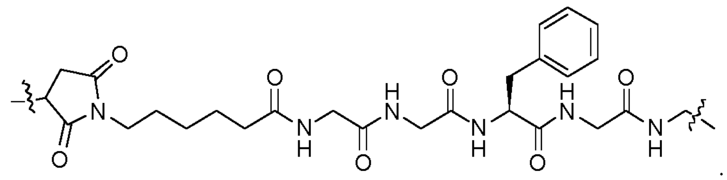

**20.** The antibody-drug conjugate of general formula (Pc-L-Y-D) or the pharmaceutically acceptable salt thereof according to claim 13, being an antibody-drug conjugate of general formula ($Pc-L_a-Y-D$) or a pharmaceutically acceptable salt thereof:

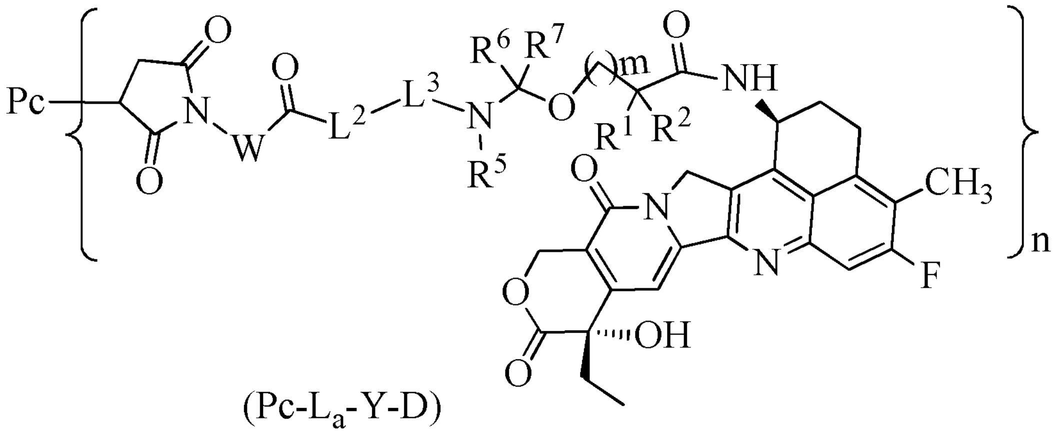

($Pc-L_a-Y-D$)

wherein,

Pc is the anti-Nectin-4 antibody according to any one of claims 1 to 8;
m is an integer from 0 to 4;
n is from 1 to 10;
$R^1$ is selected from the group consisting of halogen, haloalkyl, deuterated alkyl, cycloalkyl, cycloalkylalkyl, alkoxyalkyl, heterocyclyl, aryl and heteroaryl; $R^2$ is selected from the group consisting of hydrogen, halogen, haloalkyl, deuterated alkyl, cycloalkyl, cycloalkylalkyl, alkoxyalkyl, heterocyclyl, aryl and heteroaryl; or $R^1$ and $R^2$, together with the carbon atom to which they are attached, form cycloalkyl or heterocyclyl;
W is selected from the group consisting of $C_{1-6}$ alkylene and $C_{1-6}$ alkylene-$C_{3-6}$ cycloalkyl, wherein the $C_{1-6}$ alkylene and $C_{1-6}$ alkylene-$C_{3-6}$ cycloalkyl are each independently optionally further substituted with one or more substituents selected from the group consisting of halogen, hydroxy, cyano, amino, alkyl, chloroalkyl, deuterated alkyl, alkoxy and cycloalkyl;
$L^2$ is selected from the group consisting of $-NR^4(CH_2CH_2O)p^1CH_2CH_2C(O)-$, $-NR^4(CH_2CH_2O)p^1CH_2C(O)-$, $-S(CH_2)p^1C(O)-$ and a chemical bond, wherein $p^1$ is an integer from 1 to 20;
$L^3$ is a peptide residue consisting of 2 to 7 amino acid residues, wherein the amino acid residues are selected from the group consisting of amino acid residues formed from amino acids from phenylalanine, alanine, glycine, valine, lysine, citrulline, serine, glutamic acid and aspartic acid, and are optionally further substituted with one or more substituents selected from the group consisting of halogen, hydroxy, cyano, amino, alkyl, chloroalkyl, deuterated alkyl, alkoxy and cycloalkyl;
$R^5$ is selected from the group consisting of hydrogen, alkyl, haloalkyl, deuterated alkyl and hydroxyalkyl;
$R^6$ and $R^7$ are identical or different and are each independently selected from the group consisting of hydrogen, halogen, alkyl, haloalkyl, deuterated alkyl and hydroxyalkyl.

**21.** The antibody-drug conjugate of general formula (Pc-L-Y-D) or the pharmaceutically acceptable salt thereof according to any one of claims 13 to 20, wherein the antibody-drug conjugate is:

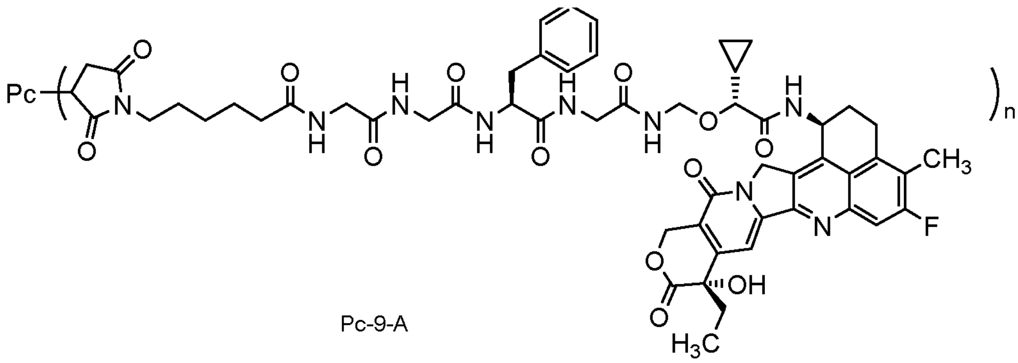

Pc-9-A

wherein:

n is from 1 to 8;
Pc is an anti-Nectin-4 antibody comprising a heavy chain set forth in SEQ ID NO: 21 and a light chain set forth in SEQ ID NO: 22.

**22.** A pharmaceutical composition comprising the anti-Nectin-4 antibody according to any one of claims 1 to 8, or the nucleic acid molecule according to claim 9, or the antibody-drug conjugate or the pharmaceutically acceptable salt thereof according to any one of claims 13 to 21, and one or more pharmaceutically acceptable excipients, diluents or carriers.

**23.** Use of the anti-Nectin-4 antibody according to any one of claims 1 to 8, or the nucleic acid molecule according to claim 9, or the antibody-drug conjugate or the pharmaceutically acceptable salt thereof according to any one of claims 13 to 21, or the pharmaceutical composition according to claim 22 in the preparation of a medicament for treating a Nectin-4-mediated disease or disorder.

**24.** Use of the anti-Nectin-4 antibody according to any one of claims 1 to 8, or the nucleic acid molecule according to claim 9, or the antibody-drug conjugate or the pharmaceutically acceptable salt thereof according to any one of claims 13 to 21, or the pharmaceutical composition according to claim 22 in the preparation of a medicament for treating and/or preventing a virus, a tumor or cancer, wherein
preferably, the tumor or cancer is selected from the group consisting of breast cancer, pancreatic cancer, lung cancer, esophageal cancer, non-small cell lung cancer, laryngeal tumors, pharyngeal tumors, oral tumors, gastric cancer, ovarian cancer, prostate cancer, bladder cancer, colorectal cancer, head and neck cancer, squamous cell carcinoma and melanoma.

**25.** A kit comprising the anti-Nectin-4 antibody according to any one of claims 1 to 8, or the nucleic acid molecule according to claim 9, or the antibody-drug conjugate or the pharmaceutically acceptable salt thereof according to any one of claims 13 to 21, or the pharmaceutical composition according to claim 22.

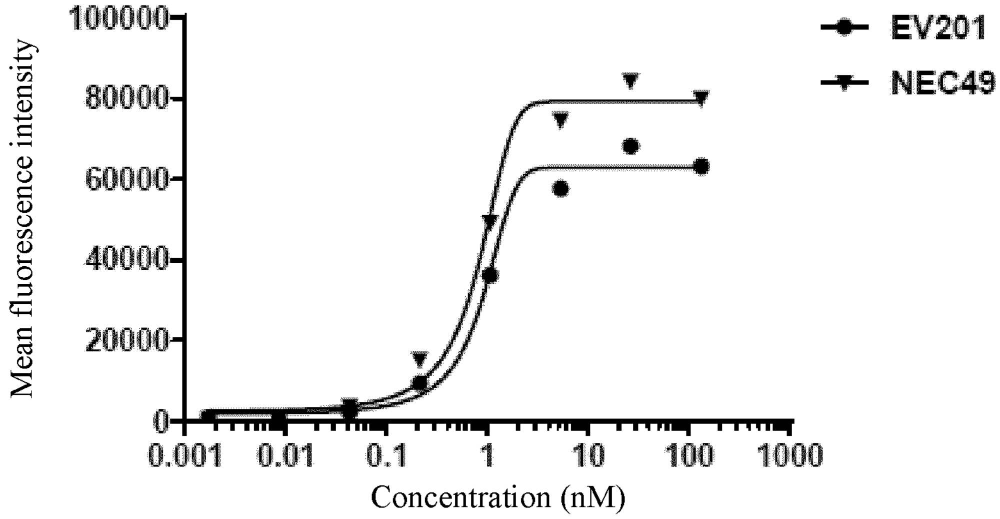
Mean fluorescence intensity
100000
80000
60000
40000
20000
0
0.001
0.01
0.1
1
10
100
1000
Concentration (nM)
EV201
NEC49

FIG. 1

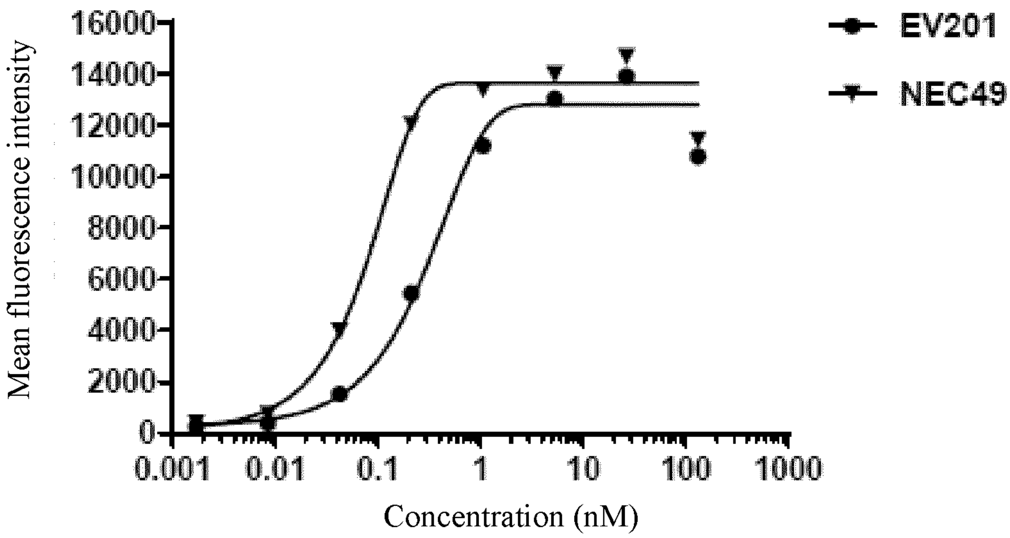
Mean fluorescence intensity
16000
14000
12000
10000
8000
6000
4000
2000
0
0.001
0.01
0.1
1
10
100
1000
Concentration (nM)
EV201
NEC49

FIG. 2

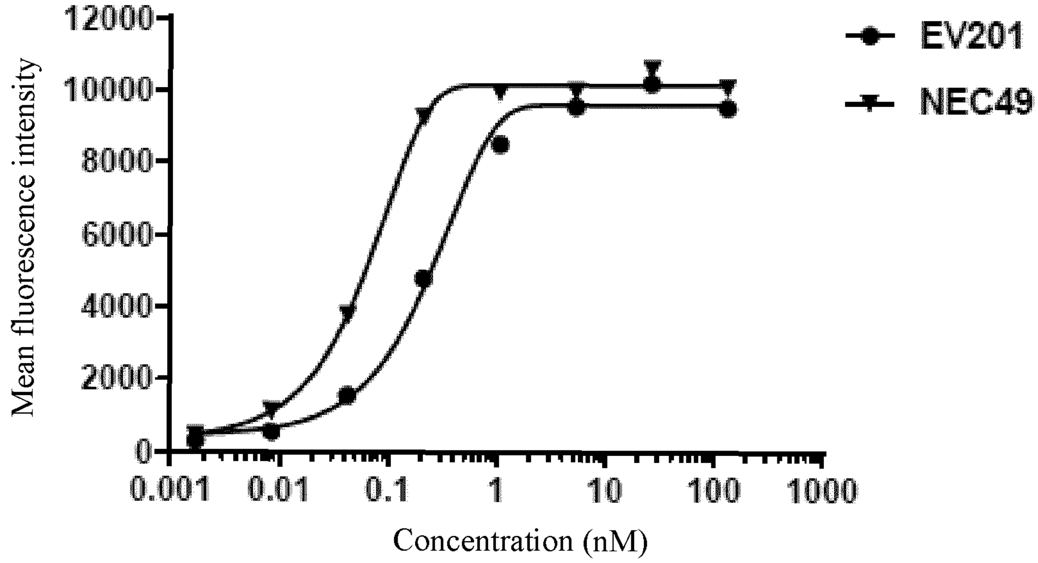
12000
10000
8000
6000
4000
2000
0
Mean fluorescence intensity
0.001
0.01
0.1
1
10
100
1000
Concentration (nM)
EV201
NEC49

FIG. 3

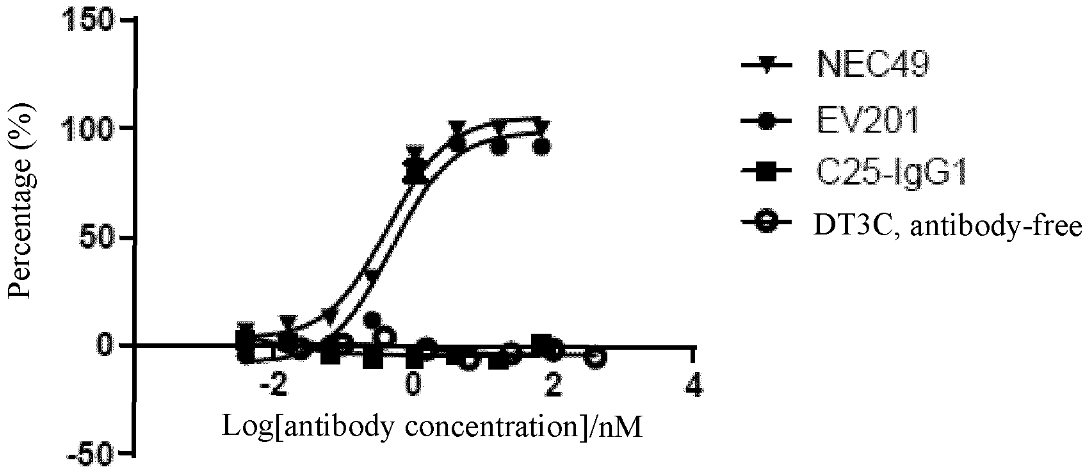
150
100
50
0
-50
Percentage (%)
-2
0
2
4
Log[antibody concentration]/nM
NEC49
EV201
C25-IgG1
DT3C, antibody-free

FIG. 4

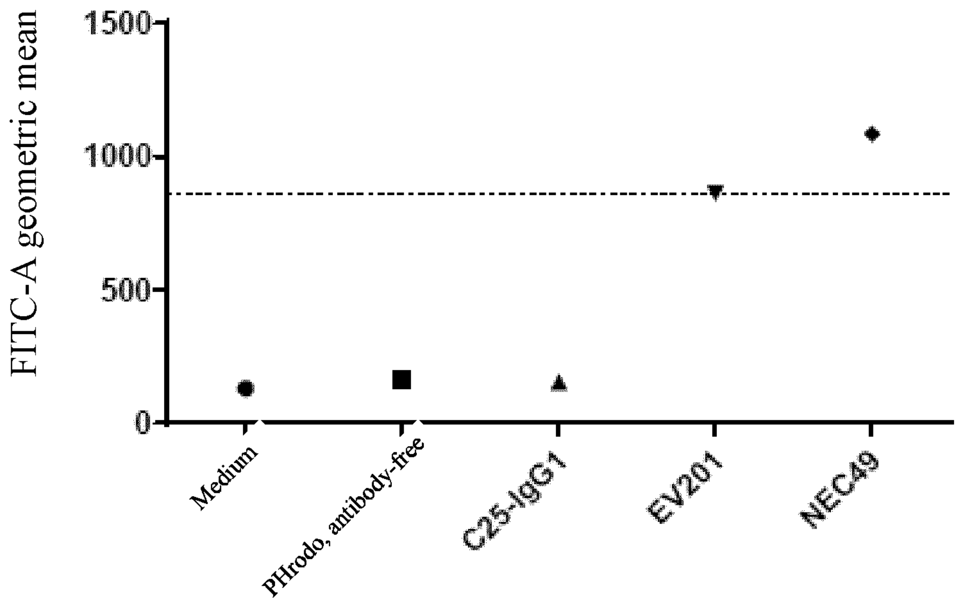
FITC-A geometric mean
1500
1000
500
0
Medium
PHrodo, antibody-free
C25-IgG1
EV201
NEC49

FIG. 5A

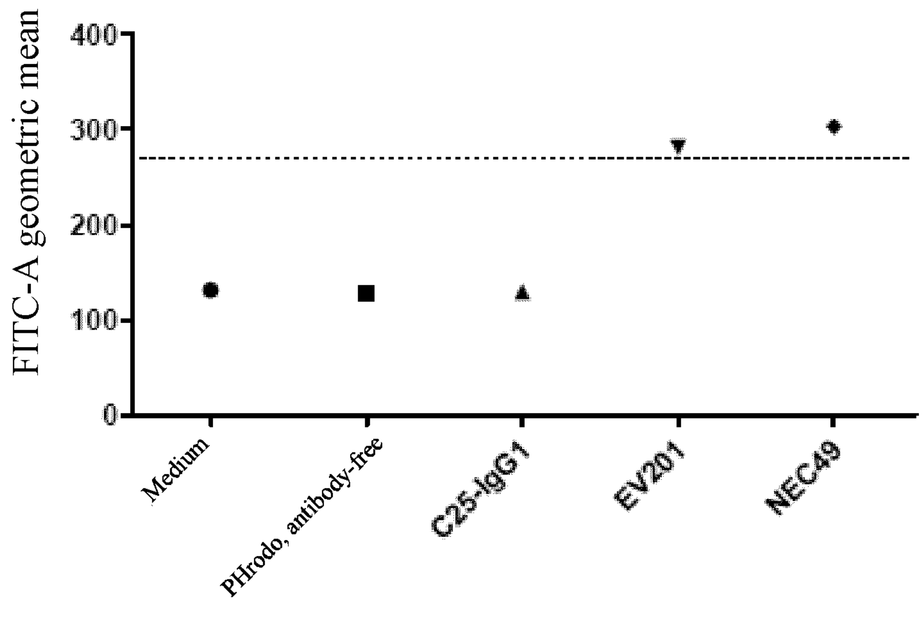
FITC-A geometric mean
400
300
200
100
0
Medium
PHrodo, antibody-free
C25-IgG1
EV201
NEC49

FIG. 5B

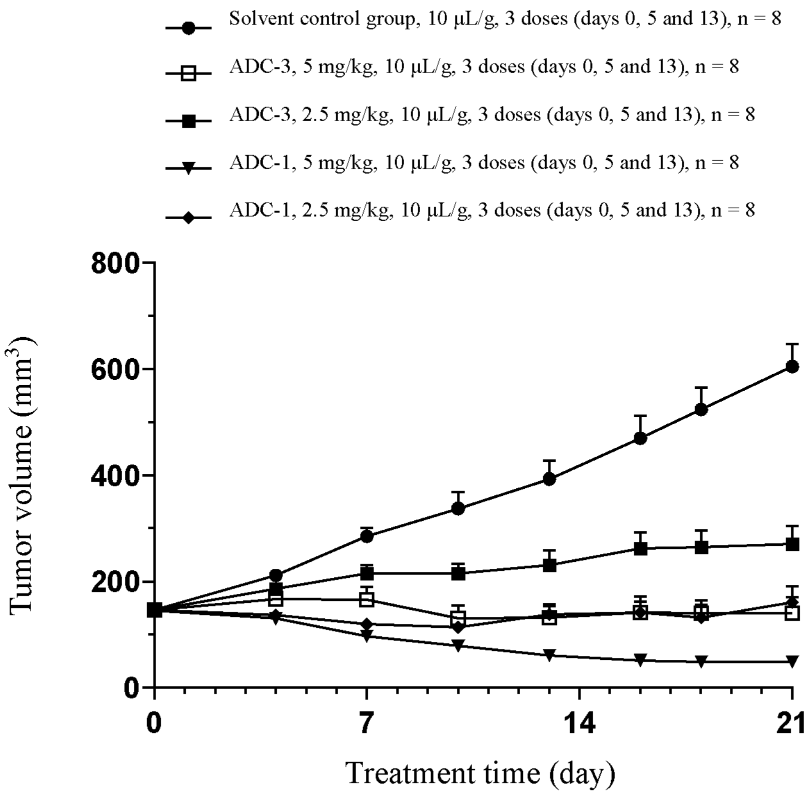
Solvent control group, 10 µL/g, 3 doses (days 0, 5 and 13), n = 8
ADC-3, 5 mg/kg, 10 µL/g, 3 doses (days 0, 5 and 13), n = 8
ADC-3, 2.5 mg/kg, 10 µL/g, 3 doses (days 0, 5 and 13), n = 8
ADC-1, 5 mg/kg, 10 µL/g, 3 doses (days 0, 5 and 13), n = 8
ADC-1, 2.5 mg/kg, 10 µL/g, 3 doses (days 0, 5 and 13), n = 8
Tumor volume (mm3)
800
600
400
200
0
0
7
14
21
Treatment time (day)

FIG. 6

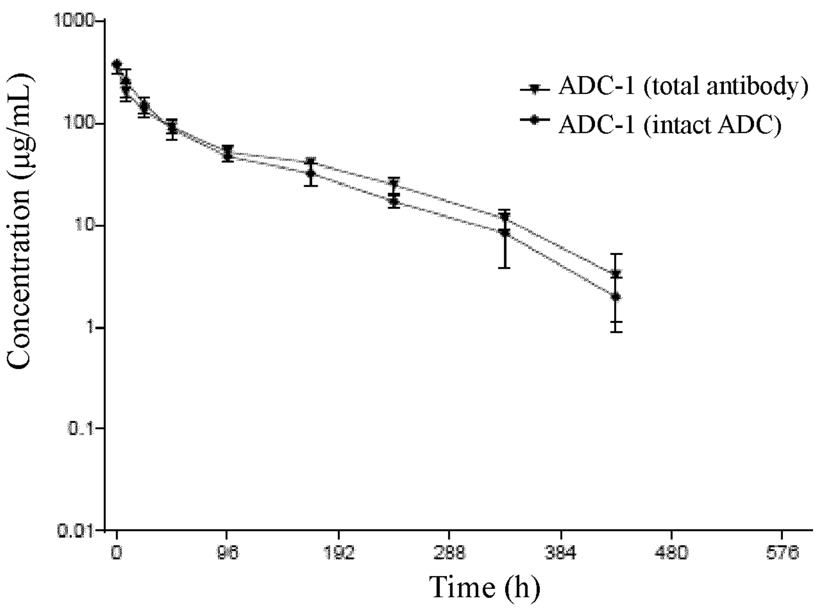
Concentration (µg/mL)
1000
100
10
1
0.1
0.01
ADC-1 (total antibody)
ADC-1 (intact ADC)
0
96
192
288
384
480
576
Time (h)

FIG. 7

## INTERNATIONAL SEARCH REPORT

International application No.
**PCT/CN2022/089129**

**A. CLASSIFICATION OF SUBJECT MATTER**

C07K 16/28(2006.01)i; C12N 15/13(2006.01)i; A61K 39/395(2006.01)i; A61K 47/68(2017.01)i; A61K 31/4745(2006.01)i; A61P 35/00(2006.01)i

According to International Patent Classification (IPC) or to both national classification and IPC

**B. FIELDS SEARCHED**

Minimum documentation searched (classification system followed by classification symbols)

C07K; C12N; A61K; A61P

Documentation searched other than minimum documentation to the extent that such documents are included in the fields searched

Electronic data base consulted during the international search (name of data base and, where practicable, search terms used)

CNTXT; WPABS; CJFD; DWPI; VEN; CNKI; PUBMED; STN; 中国专利生物序列检索系统, Chinese Patent Biological Sequence Retrieval System; 百度学术, BAIDU SCHOLAR: 江苏恒瑞, 上海恒瑞, 杨阳, 张昊颖, 抗体, antibody, mab, PVRL4, 脊髓灰质炎病毒受体样分子4, 脊髓灰质炎病毒受体相关蛋白4, nectin, nectin-4, nectin4, 偶联物, conjugate, 依喜替康, SEQ ID NOs: 6-22

**C. DOCUMENTS CONSIDERED TO BE RELEVANT**

| Category* | Citation of document, with indication, where appropriate, of the relevant passages | Relevant to claim No. |
|---|---|---|
| X | CN 110392697 A (FRENCH NATIONAL INSTITUTE OF HEALTH AND MEDICAL RESEARCH et al.) 29 October 2019 (2019-10-29)<br>claims 1-13 | 1, 4, 7-12, 22-25 |
| Y | CN 110392697 A (FRENCH NATIONAL INSTITUTE OF HEALTH AND MEDICAL RESEARCH et al.) 29 October 2019 (2019-10-29)<br>claims 1-13 | 13-25 |
| Y | WO 2020063673 A1 (JIANGSU HENGRUI MEDICINE CO., LTD. et al.) 02 April 2020 (2020-04-02)<br>claims 1, 9-20 | 13-25 |
| X | WO 2021069508 A1 (AIX-MARSEILLE UNIVERSITY et al.) 15 April 2021 (2021-04-15)<br>claims 1-16 | 1, 4, 7-12, 22-25 |
| Y | WO 2021069508 A1 (AIX-MARSEILLE UNIVERSITY et al.) 15 April 2021 (2021-04-15)<br>claims 1-16 | 13-25 |
| Y | CN 104755494 A (DAIICHI SANKYO CO., LTD.) 01 July 2015 (2015-07-01)<br>claims 1-33 | 13-25 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **13 July 2022** | **27 July 2022** |
| Name and mailing address of the ISA/CN<br>**China National Intellectual Property Administration (ISA/CN)**<br>**No. 6, Xitucheng Road, Jimenqiao, Haidian District, Beijing 100088, China**<br>Facsimile No. **(86-10)62019451** | Authorized officer<br><br>Telephone No. |

Form PCT/ISA/210 (second sheet) (January 2015)

**INTERNATIONAL SEARCH REPORT**

International application No.
**PCT/CN2022/089129**

**C. DOCUMENTS CONSIDERED TO BE RELEVANT**

| Category* | Citation of document, with indication, where appropriate, of the relevant passages | Relevant to claim No. |
|---|---|---|
| A | WO 2020244657 A1 (SHANGHAI HANSOH BIOMEDICAL CO., LTD. et al.) 10 December 2020 (2020-12-10)<br>entire document | 1-25 |
| A | WO 2020063676 A1 (JIANGSU HENGRUI MEDICINE CO., LTD. et al.) 02 April 2020 (2020-04-02)<br>entire document | 1-25 |
| A | WO 2018065501 A1 (F.HOFFMANN-LA ROCHE AG et al.) 12 April 2018 (2018-04-12)<br>entire document | 1-25 |

Form PCT/ISA/210 (second sheet) (January 2015)

**INTERNATIONAL SEARCH REPORT**

International application No.
**PCT/CN2022/089129**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   - a. ☑ forming part of the international application as filed:
     - ☑ in the form of an Annex C/ST.25 text file.
     - ☐ on paper or in the form of an image file.
   - b. ☐ furnished together with the international application under PCT Rule 13*ter*.1(a) for the purposes of international search only in the form of an Annex C/ST.25 text file.
   - c. ☐ furnished subsequent to the international filing date for the purposes of international search only:
     - ☐ in the form of an Annex C/ST.25 text file (Rule 13*ter*.1(a)).
     - ☐ on paper or in the form of an image file (Rule 13*ter*.1(b) and Administrative Instructions, Section 713).
2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.
3. Additional comments:

Form PCT/ISA/210 (continuation of first sheet) (January 2015)

**INTERNATIONAL SEARCH REPORT**
**Information on patent family members**

International application No.
**PCT/CN2022/089129**

| Patent document cited in search report | Publication date (day/month/year) | Patent family member(s) | Publication date (day/month/year) |
|---|---|---|---|
| CN 110392697 A | 29 October 2019 | EP 3589654 A1 | 08 January 2020 |
| | | JP 2020510432 A | 09 April 2020 |
| | | US 2021324104 A1 | 21 October 2021 |
| | | WO 2018158398 A1 | 07 September 2018 |
| WO 2020063673 A1 | 02 April 2020 | JP 2022512568 A | 07 February 2022 |
| | | EP 3854816 A1 | 28 July 2021 |
| | | CN 112543771 A | 23 March 2021 |
| | | TW 202028242 A | 01 August 2020 |
| | | CA 3114474 A1 | 02 April 2020 |
| | | AU 2019351427 A1 | 15 April 2021 |
| | | US 2021347894 A1 | 11 November 2021 |
| | | BR 112021004829 A2 | 08 June 2021 |
| | | KR 20210068457 A | 09 June 2021 |
| WO 2021069508 A1 | 15 April 2021 | IL 291966 D0 | 01 June 2022 |
| | | AU 2020364959 A1 | 07 April 2022 |
| | | KR 20220079614 A | 13 June 2022 |
| | | CA 3156451 A1 | 15 April 2021 |
| CN 104755494 A | 01 July 2015 | LT 2907824 T | 11 June 2018 |
| | | US 2016279259 A1 | 29 September 2016 |
| | | CA 3021435 A1 | 17 April 2014 |
| | | JP 2022008581 A | 13 January 2022 |
| | | JP 6030267 B1 | 24 November 2016 |
| | | SI 2907824 T1 | 29 June 2018 |
| | | PT 3342785 T | 25 February 2020 |
| | | NZ 746440 A | 29 November 2019 |
| | | PT 2907824 T | 07 June 2018 |
| | | SG 10201804788 X A | 30 July 2018 |
| | | JP 5953378 B2 | 20 July 2016 |
| | | US 2020282073 A1 | 10 September 2020 |
| | | ES 2773710 T3 | 14 July 2020 |
| | | CA 2885800 A1 | 17 April 2014 |
| | | WO 2014057687 A1 | 17 April 2014 |
| | | AU 2020200548 A1 | 13 February 2020 |
| | | US 2015297748 A1 | 22 October 2015 |
| | | KR 20180030734 A | 23 March 2018 |
| | | HR P20180870 T1 | 13 July 2018 |
| | | TR 201809636 T4 | 23 July 2018 |
| | | KR 20150067149 A | 17 June 2015 |
| | | KR 20190135559 A | 06 December 2019 |
| | | CY 1120363 T1 | 10 July 2019 |
| | | KR 20180105271 A | 27 September 2018 |
| | | SG 11201502887 W A | 28 May 2015 |
| | | TW 202033499 A | 16 September 2020 |
| | | PL 3342785 T3 | 07 September 2020 |
| | | RS 57278 B1 | 31 August 2018 |
| | | EP 3632471 A1 | 08 April 2020 |
| | | TW 201811746 A | 01 April 2018 |
| | | KR 20220029776 A | 08 March 2022 |
| | | DK 3342785 T3 | 23 March 2020 |
| | | TW 201420117 A | 01 June 2014 |

Form PCT/ISA/210 (patent family annex) (January 2015)

**INTERNATIONAL SEARCH REPORT**
**Information on patent family members**

International application No.
**PCT/CN2022/089129**

| Patent document cited in search report | Publication date (day/month/year) | Patent family member(s) | Publication date (day/month/year) |
|---|---|---|---|
| | | KR 20210132240 A | 03 November 2021 |
| | | PH 12018501433 A1 | 27 February 2019 |
| | | ZA 201501825 B | 25 May 2022 |
| | | DK 2907824 T3 | 23 July 2018 |
| | | PL 2907824 T3 | 31 August 2018 |
| | | IL 238143 D0 | 31 May 2015 |
| | | HK 1256631 A1 | 27 September 2019 |
| | | KR 20210038728 A | 07 April 2021 |
| | | CN 113929738 A | 14 January 2022 |
| | | KR 20200026323 A | 10 March 2020 |
| | | SI 3342785 T1 | 28 February 2020 |
| | | JP 2016196484 A | 24 November 2016 |
| | | NZ 705394 A | 26 October 2018 |
| | | EP 2907824 A1 | 19 August 2015 |
| | | IL 271760 D0 | 27 February 2020 |
| | | RS 60000 B1 | 30 April 2020 |
| WO 2020244657 A1 | 10 December 2020 | EP 3981434 A1 | 13 April 2022 |
| | | AU 2020288275 A1 | 03 February 2022 |
| | | KR 20220017946 A | 14 February 2022 |
| | | BR 112021024406 A2 | 19 April 2022 |
| | | TW 202112398 A | 01 April 2021 |
| | | CA 3142641 A1 | 10 December 2020 |
| WO 2020063676 A1 | 02 April 2020 | CA 3114137 A1 | 02 April 2020 |
| | | JP 2022511351 A | 31 January 2022 |
| | | BR 112021004656 A2 | 01 June 2021 |
| | | EP 3858386 A1 | 04 August 2021 |
| | | KR 20210066833 A | 07 June 2021 |
| | | US 2021353764 A1 | 18 November 2021 |
| | | AU 2019349207 A1 | 08 April 2021 |
| | | TW 202027795 A | 01 August 2020 |
| | | CN 112512591 A | 16 March 2021 |
| WO 2018065501 A1 | 12 April 2018 | JP 2019537558 A | 26 December 2019 |
| | | CN 110139674 A | 16 August 2019 |
| | | EP 3522933 A1 | 14 August 2019 |
| | | US 2019201544 A1 | 04 July 2019 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2012047724 A
- US 4816567 A
- US 5500362 A
- US 5821337 A
- US 20050238649 A1
- US 5208020 A
- WO 2020063676 A1
- WO 2020063676 A


### Non-patent literature cited in the description


- *J. biol. chem,* 1968, vol. 243, 3558
- **KABAT et al.** Sequences of Proteins of Immunological Interest. Public Health Service, National Institutes of Health, 1991
- **MARTIN, ACR.** *Protein Sequence and Structure Analysis of Antibody Variable Domains[J,* 2001
- **LEFRANC, M.P. et al.** *Dev. Comp. Immunol.,* 2003, vol. 27, 55-77
- *Front Immunol.,* 16 October 2018, vol. 9, 2278
- **MARKS et al.** *Bio/Technology,* 1992, vol. 10, 779-783
- **BARBAS et al.** *PNAS,* 1994, vol. 91, 3809-3813
- **SCHIER et al.** *Gene,* 1995, vol. 169, 147-155
- **YELTON et al.** *J.Immunol.,* 1995, vol. 155, 1994-2004
- **JACKSON et al.** *J.Immunol.,* 1995, vol. 154 (7), 3310-9
- **HAWKINS et al.** *J. Mol. Biol.,* 1992, vol. 226, 889-896
- **KOHLER et al.** *Nature,* 1975, vol. 256, 495
- **CLACKSON et al.** *Nature,* 1991, vol. 352, 624-628
- **MARKS et al.** *J. Mol. Biol.,* 1991, vol. 222, 581-597
- **PRESTA.** *J. Allergy Clin. Immunol.,* 2005, vol. 116, 731
- *Meth. Mol. Biol.,* 2004, vol. 248, 443-463
- *Prot. Sci.,* 2000, vol. 9, 487-496
- **HARLOW ; LANE.** Antibodies. Cold Spring Harbor Press
- **JUNGHANS et al.** *Cancer Res.,* 1990, vol. 50, 1495-1502
- **RAVETCH ; KINET.** *Annu. Rev. Immunol,* 1991, vol. 9, 457-92
- **CLYNES et al.** *USA,* 1998, vol. 95, 652-656
- **BATZER et al.** *Nucleic Acid Res.,* 1991, vol. 19, 5081
- **OHTSUKA et al.** *J. Biol. Chem.,* 1985, vol. 260, 2605-2608
- **ROSSOLINI et al.** *Mol. Cell. Probes,* 1994, vol. 8, 91-98
- **WATSON et al.** Molecular Biology of the Gene. The Benjamin/Cummings Pub. Co, 1987, 224
- Antibodies: A Laboratory Manual. Cold Spring Harbor Press
- *immunoglobulin journal,* 2001, ISBN 012441351
- **CHARI et al.** *Cancer Research,* 1992, vol. 52, 127-131
- **SAMBROOK et al.** Molecular Cloning: A Laboratory Manual. Cold Spring Harbor Laboratory Press
- **AUSUBEL et al.** Current Protocols in Molecular Biology. Greene Publishing Association, Wiley Interscience
- *CHEMICAL ABSTRACTS,* 646502-53-6